# EUROPEAN PATENT SPECIFICATION

(11) **EP 4 362 953 B1**
(45) Date of publication and mention of the grant of the patent: **03.12.2025**
(21) Application number: 22738430.2
(22) Date of filing: 27.06.2022
(51) Int. Cl.: A61K 31/65, A61P 31/12, A61P 31/14, A61P 31/16

(54) **TETRACYCLINE DERIVATIVE-INDUCED MITOHORMESIS MEDIATES DISEASE TOLERANCE AGAINST VIRAL INFECTIONS**
TETRACYCLINDERIVAT-INDUZIERTE MITOHORMESE VERMITTELT KRANKHEITSTOLERANZ GEGEN VIRALE INFEKTIONEN
MITOHORMÈSE INDUITE PAR UN DÉRIVÉ DE LA TÉTRACYCLINE MODIFIANT LA TOLÉRANCE À LA MALADIE CONTRE LES INFECTIONS VIRALES

(30) Priority: 28.06.2021 EP 21182100
(43) Date of publication of application: 08.05.2024
(73) Proprietor: ECOLE POLYTECHNIQUE FEDERALE DE LAUSANNE (EPFL), 1015 Lausanne (CH)
(72) Inventor: AUWERX, Johan, 1164 Buchillon (CH); MOTTIS, Adrienne, 1806 Saint-Légier-La-Chiésaz (CH); NELSON, Mark, Midway, Utah 84049 (US)
(74) Representative: Vossius & Partner Patentanwälte Rechtsanwälte mbB
(86) International application number: PCT/EP2022/067538
(87) International publication number: WO 2023/274941

(56) References cited:
- WO-A1-2005/082860
- CHEN I-J ET AL: "Identification of HIV-1 integrase inhibitors via three-dimensional database searching using ASV and HIV-1 integrases as targets", BIOORGANIC, ELSEVIER, AMSTERDAM, NL, vol. 8, no. 10, 1 October 2000 (2000-10-01), pages 2385 - 2398, XP002680740, ISSN: 0968-0896, DOI: 10.1016/S0968-0896(00)00180-2

## Description

The present invention relates to a compound of formula (I) or formula (II) 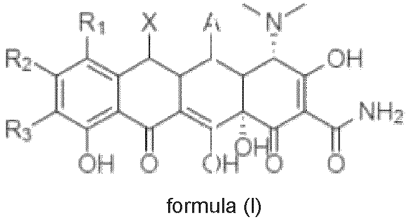 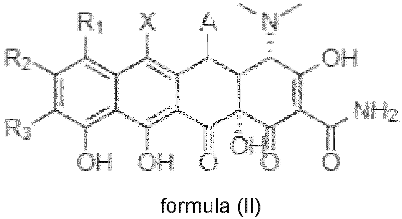 or a pharmaceutically acceptable salt thereof for use in treating or preventing a respiratory viral disease or viral infection, wherein within formula (I) or (II) (A) A is H or OH, X is CH₃, and R₁ and R₂ are H and R₃ is efa linear or branched C3 to C8 alkyl; or (B) A is H or OH, X is CH₃, and R₂ is H and R₁ and R₃ are a heteroaryl.

Any subject-matter outside the scope of the claims does not form part of the invention and is provided for reference or comparison only. are independently selected from the group consisting of a linear or branched C3 to C8 alkyl, a cycloalkyl, an alkenyl, a phenylalkenyl (preferably styryl), an aryl, and a heteroaryl; or (C) A is H or OH, X is H or CH₃, and R₁ to R₃ are each independently selected from H, F, Cl, Br, or I, provided that at least one of R₁ to R₃ is F, Cl, Br, or I; or wherein within formula (II) (D) A is H or OH, X is CH₃, and R₁ to R₃ are H, and wherein for (A) and (B) the heteroatom of the heteroaryl is selected from S, O and N, and the linear or branched C3 to C8 alkyl, cycloalkyl, alkenyl, phenylalkenyl (preferably styryl), aryl, and heteroaryl are unsubstituted or are substituted, preferably substituted with one or more of F, CI, Br, I, -OH, -SH, -NH₂, -N₃, -NO₂, - CN, -CHO, -COOH, or -CONH₂ and more preferably with one or more F or Br.

In this specification, a number of documents including patent applications and manufacturer's manuals are cited.

WO 2005/082860 concerns the use of calpain inhibitors and discloses that calpains are involved in viral diseases and block the replication of HIV-1 and severe acute respiratory syndrome-associated coronavirus (SARS-CoV). The document further discloses the compounds 7,9-Dibromosancycline, 7-Chlorosancycline, 7-Bromosancycline and 7-lodosancycline as active compounds.

Cells constantly monitor the function of their mitochondria and activate adaptive mitochondrial stress responses (MSR) to maintain or restore mitochondrial homeostasis upon stress. Mitohormesis is the phenomenon that ensues when these adaptive responses surpass the initial stress and lead to overall beneficial consequences on cellular and organismal fitness. A prototypical and well-studied form of the MSR is the mitochondrial unfolded protein response (UPR^{mt}), first described in mammalian cells (1), but more extensively characterized in *Caenorhabditis elegans* (*C. elegans*) (reviewed in (2-4)). Fitting with a beneficial health impact, the mitohormetic induction of the MSR is reported to extend health- and lifespan in *C. elegans* (5, 6), as well as to attenuate the phenotypic consequences of Alzheimer's disease and exert cardioprotective effects in mouse models (7, 8). Interestingly, Tetracyclines (Tets) - antibiotics that not only block bacterial, but also mitochondrial translation - can be used to induce such a mild proteotoxic mitochondrial stress. Tets are therefore pharmacological tools that induce the MSR (7, 9), often resulting in a beneficial mitohormetic response.

Mitochondrial function and immunity, both innate and adaptive, are interconnected at multiple levels (10, 11). Mitochondrial metabolism is a central determinant of the type and course of immune response, and damaged mitochondria contribute to inflammation by the release of damage-associated molecular patterns (DAMPs) amongst other mechanisms (*12*). On top, mitochondria can be targeted by multiple bacterial as well as viral infections (*13*). Mitochondrial function has thus been proposed to be essential to trigger tolerance to infection (*14, 15*). Resistant hosts fight infection by eliciting an immune response that reduces pathogen load, whereas tolerance refers to the mechanisms that limit the extents of organ dysfunction and tissue damage caused by infection without impacting on pathogen load (*16*)*.*

Respiratory viruses such as Influenza A virus (IFV) or SARS-CoV2 represent a major public health concern as our aging population is highly susceptible to the complications and often lethal consequences of such infections (*17*). Uncontrolled systemic inflammation ensuing from infection by respiratory viruses can lead to acute respiratory distress syndrome (ARDS) and multiorgan dysfunction syndrome (MODS), which are both in part driven by mitochondrial dysfunction (*18, 19*), contributing significantly to complications and mortality.

Because the ability of the immune system of humans and other mammals to thwart viral infections is limited, current anti-viral treatments are suboptimal, and viral infections lead to a significant comorbidities or even mortality that cost the health care system and veterinary medicine system billions of dollars annually worldwide, there is an ongoing need for novel means for treating and preventing viral infections. This need is addressed by the present application.

Accordingly the present invention relates to a compound of formula (I) or formula (II) 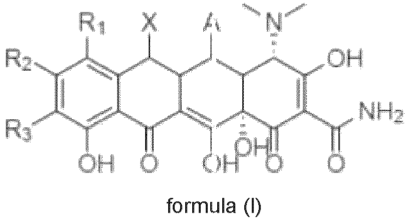 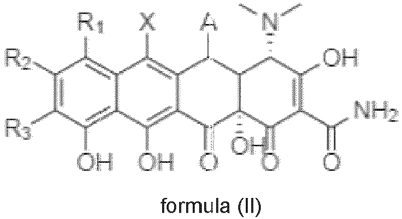 or a pharmaceutically acceptable salt thereof for use in treating or preventing a respiratory viral disease or viral infection, wherein within formula (I) or (II) (A) A is H or OH, X is CH₃, and R₁ and R₂ are H and R₃ is a linear or branched C3 to C8 alkyl; or (B) A is H or OH, X is CH₃, and R₂ is H and R₁ and R₃ a heteroaryl.

The subject to be treated is preferably a mammal such as a mouse, rat, swine, bovines or monkey, and is most preferably a human.

A subject has a viral infection if the body of the subject is invaded by infectious virus particles (virions). Virions are capable of entering susceptible cells within the subject. A subject having a viral infection does not necessarily have a viral disease, i.e. have the disease symptoms that may be caused by the viral infection. For instance, in the case of the SARS-CoV2 virus a subject can be infected by the virus but may be asymptomatic and may not have COVID-19, noting that COVID-19 is the disease being caused by the SARS-CoV2 virus and is often characterized by one or more of fever, cough, headache, fatigue, breathing difficulties, and loss of smell and taste. An infected but asymptomatic subject may nevertheless spread the virus and, thus, should also be treated.

Hence, a viral infection is the presence of a harmful virus and generally also the proliferation of the virus in the body of a subject. Viruses cannot reproduce without the assistance of a host. Viruses infect a host by introducing their genetic material into the cells and hijacking the cell's internal machinery to make more virus particles. A viral disease is any condition that is caused by the viral infection. In the context of the present invention, viral diseases and infections are limited to respiratory viral diseases and infections.

The salt of the compound according to the invention is not particularly limited and may be any salt as long as it is pharmaceutically acceptable. The term "pharmaceutically acceptable salts" is art recognized and includes relatively non-toxic, inorganic and organic acid addition salts of compounds according to the present invention. These salts can be prepared *in situ* during the final isolation and purification of the compounds according to the invention, or by separately reacting a purified compound according the invention in its free base form with a suitable organic or inorganic acid, and isolating the salt thus formed. Representative salts include the hydrobromide, hydrochloride, sulfate, bisulfate, phosphate, nitrate, acetate, valerate, oleate, palmitate, stearate, laurate, benzoate, lactate, phosphate, tosylate, citrate, maleate, fumarate, succinate, tartrate, napthylate, mesylate, glucoheptonate, lactobionate, and laurylsulphonate salts and the like (See, e.g., Berge et al. (1977) "Pharmaceutical Salts", J. Farm. SCI 66:1-19). Preferred examples are a calcium salt, a maleate, an oxalate, and a hyclate (also known as hydrochloride hemiethanolate hemihydrate).

Among the compounds complying with the requirements of items (A) to (D), items (A) to (C) are preferred, items (A) and (B) are more preferred and item (A) is most preferred. As will be further discussed herein below, compounds complying with the requirements of items (A) to (C) display a significantly reduced antibacterial activity (as determined by the minimum inhibitory concentration) as compared to doxycycline (Dox). In addition and as will as be discussed in greater detail herein below, compounds complying with the requirements of item (A) outstandingly well activate an adaptive mitochondrial stress response. However, only items (A) and (B) as defined in the claims are part of the invention.

The compounds of (A) to (C) may be based on formula (I) or (II) while the compounds of (D) are solely based on formula (II) and are not based on formula (I). The compounds of (A) to (C) are preferably based on formula (I).

The compounds complying with the requirements of item (A) are based on the exemplified compound 9-t-butyl-doxycycline (9-TB). The compounds complying with the requirements of item (B) are based on the exemplified compound 7,9-bis3POyr26 di, F dox. The compounds complying with the requirements of item (C) are based on the exemplified compounds 7-bromo sancycline and 8-bromo sancycline, and are not part of the present invention.

Finally, the compounds complying with the requirements of item (D) are based on the exemplified compound anhydro-tetracycline (ATc), and are not part of the present invention either.

Moreover, Markush formula (I) is based on tetracycline while Markush formula (II) is based on sancycline (also known as 6-demethyl-6-deoxytetracycline; bonomycin; or norcycline). Sancycline is a semisynthetic tetracycline and can be prepared by hydrogenolysis of the chloro and benzylic hydroxy moieties of declomycin. Like other tetracycline derivatives, sancycline acts as an antibiotic by reversibly binding to the 30S ribosomal subunit and inhibiting protein translation by blocking entry of aminoacyl-tRNA into the ribosome A site.

The (substituted or unsubstituted) heteroaryl is preferably a (substituted or unsubstituted) C6 to C10 heteroaryl, is more preferably a (substituted or unsubstituted) C5 or C6 heteroaryl aryl and is most preferably (substituted or unsubstituted) pyridine. The number of heteroatoms of the (substituted or unsubstituted) heteroaryl is preferably 1, 2 or 3, more preferably 1 or 2 and most preferably 1.

With the list of the possible substituents of F, Cl, Br, I, -OH, -SH, -NH₂, -N₃, -NO₂, -CN, -CHO, -COOH, or -CONH₂, (i) F, Cl, Br, I, -OH, -SH, and -NH₂ are preferred, (ii) F, Cl, Br, and I are more preferred, and (iii) F or Br are most preferred.

The compound according to the invention may be formulated as a pharmaceutical composition. In accordance with the present invention, the term "pharmaceutical composition" relates to a composition for administration to a subject having a respiratory disease or viral infection. The pharmaceutical composition of the invention comprises one or more of the compounds recited above. It may, optionally, comprise further molecules capable of altering the characteristics of the compounds according to the invention thereby, for example, stabilizing, modulating and/or activating their function. The composition may be in solid, liquid or gaseous form and may be, inter alia, in the form of (a) powder(s), (a) capsules(s), (a) tablet(s), (a) solution(s) or (an) aerosol(s). Capsule(s) and tablet(s) are preferred.

The pharmaceutical composition according to the present invention may, optionally and additionally, comprise a pharmaceutically acceptable carrier. Examples of suitable pharmaceutical carriers are well known in the art and include phosphate buffered saline solutions, water, emulsions, such as oil/water emulsions, various types of wetting agents, sterile solutions, organic solvents including DMSO etc. Compositions comprising such carriers can be formulated by well-known conventional methods. These pharmaceutical compositions can be administered to the subject at a suitable dose. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. The therapeutically effective amount for a given situation will readily be determined by routine experimentation and is within the skills and judgement of the ordinary clinician or physician. Preferred examples of dosages and regimens based on the active compound according to the invention will be described herein below.

The mode of administration is preferably selected from oral administration, intrapleural instillation, intraperitoneal injection, intravenous injection and intravenous infusion. Oral administration and intravenous infusion are most preferred.

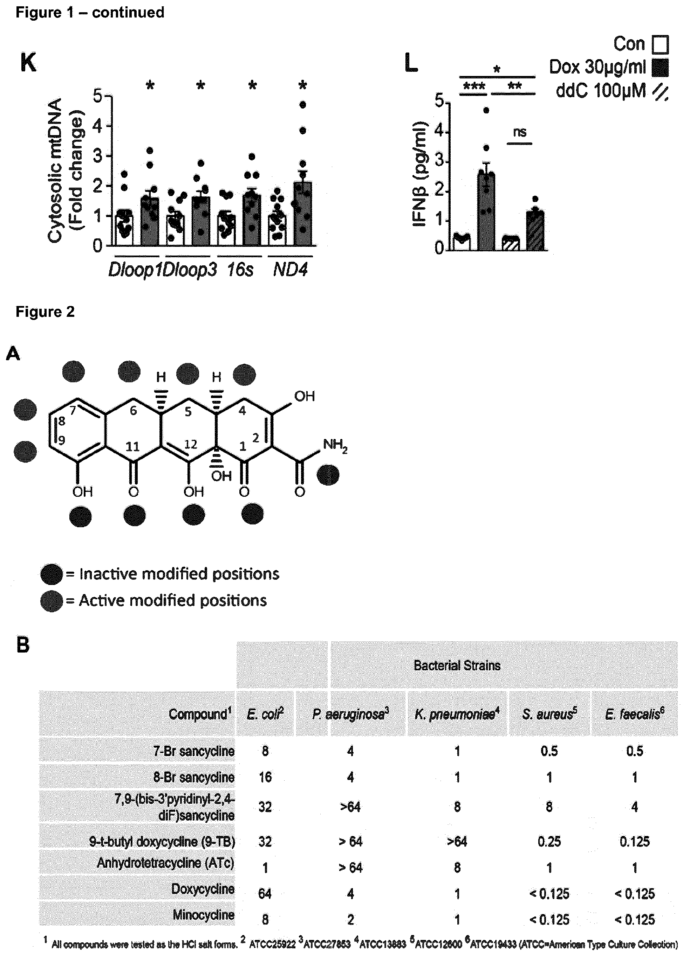

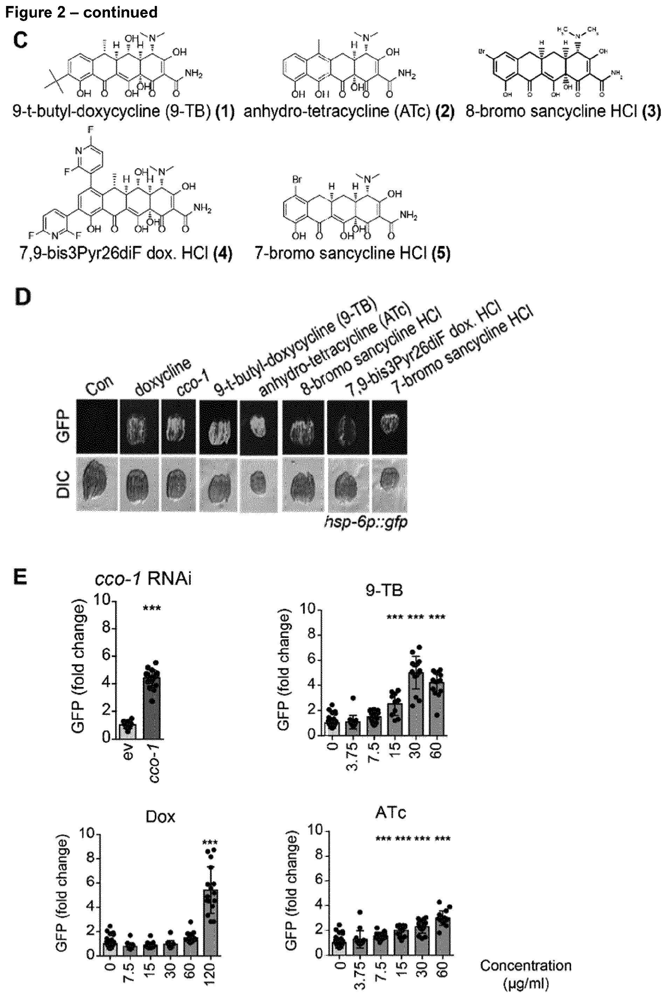

In the examples herein below the potential of 51 tetracycline derivatives (Fig 2. and Table 1) to induce MSR and in particular mitohormesis has been investigated. Examples concerning compounds outside the scope of the claims are provided for reference and/or comparison only. 9-t-butyl-doxycycline (9-TB), 7,9-bis3POyr26 diF dox, 7-bromo sancycline, 8-bromo sancycline and anhydro-tetracycline (ATc) were found to strongly induce MSR and in particular mitohormesis. 9-TB is the strongest inducer of MSR. In a mouse model it was surprisingly found that these compounds induce tolerance and survival in the context of a respiratory infection caused by an otherwise lethal dose of influenza A. The compounds are shown to lower systemic and local inflammation and limit lung tissue damage. Bioinformatic analysis of blood cell types of humans infected with SARS-CoV2 indicates that the MSR, as induced by tetracycline derivatives according to invention, are protective against viral infection and disease in general.

It is important to note that this therapeutic effect is not based on an antibiotic effect of the compound according to the invention but on mitohormesis and tolerance induction. This is evidenced by the transcriptomic response to the compounds in germ-free mice, and the fact that the induced MSR crosstalks with the innate immune system, in particular with type I interferon (IFN) signaling.

The compounds 9-t-butyl-doxycycline (9-TB), 7,9-bis3POyr26 diF dox, 7-bromo sancycline, 8-bromo sancycline and certainly also the related compounds as defined in the above items (A) to (C) offer the additional advantage that they are essentially devoid of antibacterial activity but still very potently trigger the MSR (as evidenced in worms and human cells). In this respect 9-t-butyl-doxycycline (9-TB) was most potent. This avoids unwanted side effects by antimicrobial activity since anti-microbial tetracyclines interfere with the host microbiome such as the intestinal flora.

It is known from the prior art that chemical modifications along the upper periphery, positions C5-C9, of tetracycline derivatives influence antibacterial activity, depending upon the position and nature of the chemical substitution, and derivatives at these positions have produced clinically significant antibiotics, particularly at the C9 positions, resulting in the recent 3rd generation semisynthetic antibiotics Tygacil^{®}, Nuzyra^{®}, Seysara^{®}, and the totally synthetic tetracycline Eravacycline^{®}. As new 3rd generation antibiotics they also possess varying bioactivities including activity against mammalian cells studied in inflammation states, cancer and neurodegeneration. It has been surprisingly found herein that modifying positions C7-C9 and hence the exactly same region of the naphthacene ring of tetracycline derivatives, results in the case of the discussed compounds according to the invention in compounds being essentially devoid of antibacterial activity but still very potently triggering MSR.

Solely the MSR and in particular mitohormesis as induced by the compounds according to the invention was unexpectedly found to improve survival and disease tolerance against lethal influenza virus infection. This capability of the particular tetracycline derivative according to the invention was to the best knowledge of the inventors neither disclosed nor suggested by the prior art.

Accordingly, the purpose of the invention of "treating or preventing a respiratory viral disease or viral infection" is preferably further defined by the previously unknown medical effect being conveyed by the compound according to the invention as "treating or preventing a respiratory viral disease or viral infection by activating MSR" or as "treating or preventing a respiratory viral disease or viral infection by inducing mitohormesis" or as "treating or preventing a respiratory viral disease or viral infection by inducing endogenous interferon production through mitohormesis".

All these medical effects result in a particular clinical situation because, as explained, the compounds according to the invention do not directly act on the virus but instead help the immune system to defeat the virus by activating MSR, in particular mitohormesis which activation results *inter alia* in the induction of endogenous interferon production.

Endogenous IFN production is the natural response of a subject to a viral infection and this natural response is boosted by the compounds according to the invention, surprisingly to an extent that protects mice from an otherwise lethal dosage of influenza A.

In accordance with a preferred embodiment of a compound according to the above item (A) of the invention R₃ is a branched C3 to C8 alkyl.

In accordance with a more preferred embodiment of the invention R₃ is isopropyl, t-butyl, or t-pentyl, and is preferably t-butyl.

The above preferred embodiment and more preferred embodiment structurally more closely resemble the exemplified compound 9-t-butyl-doxycycline (9-TB). In connection with the above preferred embodiment and more preferred embodiment the compound is most preferably 9-TB or a pharmaceutically acceptable salt thereof.

In accordance with a preferred embodiment of a compound according to the above item (B) of the invention R₁ and R₃ are a heteroaryl, wherein the heteroatom is preferably N and/or the heteroaryl is preferably substituted with one or two F.

In accordance with a more preferred embodiment of the invention R₁ and R₃ are both bis-3-pyridine-2,6-di-fluor.

The above preferred embodiment and more preferred embodiment structurally more closely resemble the exemplified compound 7,9-bis3POyr26 diF dox. In connection with the above preferred embodiment and more preferred embodiment the compound is most preferably 7,9-bis3POyr26 diF dox or a pharmaceutically acceptable salt thereof.

In accordance with a more preferred embodiment of the invention the viral respiratory viral disease or viral infections is caused by an influenza virus, coronavirus, bocavirus, rhinovirus, metapneumovirus, respiratory syncytial virus (RSV), parainfluenza viruses or respiratory adenoviruses.

In accordance with an even more preferred embodiment of the invention the influenza virus is an influenza A or B and is preferably an influenza A virus.

In accordance with another even more preferred embodiment of the invention the coronavirus is a betacoronavirus.

In accordance with a most preferred embodiment of the invention the betacoronavirus is selected from SARS-CoV-2, MERS-CoV, SARS-CoV-1, OC43, and HKU1, and is most preferably SARS-CoV-2.

In the examples herein below it is demonstrated that the compound of the invention improve survival and disease tolerance in mice against an otherwise lethal influenza virus (IFV) infection. Bioinformatic analysis of data sets of different blood cell types of humans infected with SARS-CoV2, furthermore establish that the robust relationship between the MSR and IFN signaling can be extended across species and during viral infections beyond those caused by IFV.

In view of the data on influenza virus and SARS-CoV2 in the examples the viral disease or viral infection is a respiratory viral disease or viral infection. Preferred examples of respiratory viruses are influenza virus, coronavirus, bocavirus, rhinovirus, metapneumovirus, respiratory syncytial virus (RSV), parainfluenza viruses or respiratory adenoviruses. Infections with these viruses can be found in humans.

Influenza A and B are the two types of influenza that cause epidemic seasonal infections nearly every year. Influenza A can be found in many species, including humans, birds, and pigs. Due to the breadth of potential hosts and its ability to genetically change over a short amount of time, influenza A viruses are very diverse. and can cause a pandemic. This may happen in particular when a new influenza A strains emerges that significantly differs from circulating influenza A strains. Influenza B is typically only found in humans.

SARS-CoV2 (severe acute respiratory syndrome coronavirus 2) is a coronavirus and more precisely a betacoronavirus. As of June 2021, the global spread of SARS-CoV2 has resulted in over about 175 million confirmed cases and about 3.8 million deaths attributed to COVID-19 [Johns Hopkins Center for Systems Science and Engineering (CSSE) COVID tracker]. While the SARS-CoV-2 pandemic could be slowed by restrictive isolation measures, these place an enormous burden on societies and economies, and, once lifted, exponential spread resumes. Other betacoronaviruses that can be found in humans are MERS-CoV, SARS-CoV-1, OC43, and HKU1.

The compounds according to the invention may also be used to treat or prevent zoonotic viral infections and diseases, such as diseases and infections caused by zoonotic influenza (zoonotic influenza A viruses), salmonellosis (Salmonella species), west nile virus (Flaviviridae, Flavivirus), plague (Yersinia pestis), rabies (Rhabdoviridae, Lyssavirus), brucellosis (Brucella species) and lyme disease (Borrelia burgdorferi).

Still further the compounds according to the invention may be used to treat or prevent diseases and infections caused by veterinary viruses, such as rhabdoviruses, foot and mouth disease virus, pestiviruses, arteriviruses, coronaviruses, toroviruses, Influenza (avian), Influenza (swine), bluetongue viruses, circoviruses, herpesviruses, African swine fever virus, retroviruses, flavivirus, paramyxoviruses, parvoviruses, and bat virome viruses.

In accordance with a preferred embodiment of the invention the compound is to be administered at a dosage of 0,0001 to 400 mg per kilogram of body weight per day and/or 1 to 800 mg per day.

It is shown in the examples herein below in mice that 9-tert-butyl-doxycycline (9-TB) and anhydrotetracycline (ATc) are capable of activating an adaptive mitochondrial stress response at a dose being significantly lower than the dose required for Doxycycline (Dox). In mice lower doses of 9-TB (1.88 µg/ml) 191 and ATc (3.75 and 7.5 µg/ml) were even superior as compared to higher dosages Dox (at 7.5-15 µg/ml) for activating an adaptive mitochondrial stress response.

In general, a suitable daily dose of a compound according to the invention is an amount of the compound which is the lowest dose effective to produce the therapeutic effect of treating or preventing a viral disease or viral infection. If desired, the effective daily dose of the active compound may be administered as two, three, four, five, six or more sub-doses administered separately at appropriate intervals throughout the day, optionally, in unit dosage forms.

Generally, daily doses of the compounds of this invention for a patient will range from about 0.0001 to about 400 mg per kilogram of body weight per day, more preferably from about 0.01 to about 300 mg per kg per day, and still more preferably from about 1.0 to about 200 mg per kg per day. The upper end of each of these ranges is with increasing preference 100 mg per kg per day, 50 mg per kg per day, 25 mg per kg per day and 10 mg per kg per day.

These dosages mg per kg per day are particularly suitable for intravenous and subcutaneous administration. Since for humans Dox is commonly used as antibiotic for treating bacterial infection at a dosage of 100 mg twice daily when given orally per day it is expected that a compound according to the invention can be used at a dosage as high as 400 mg per subject per day and even as high as 800, 700, 500, or 500 mg per day, preferably as high as 300 mg per day and more preferably as high as high 200 mg day and most preferably as high as high as 100 mg per day without unacceptable side effect of overdosing in humans. Hence, with increasing preferences also dosage ranges of 0.01 mg per day to 300 mg per day when given orally, 0.01 mg per day to 200 mg per day and 1 mg per day to 100 mg per day are contemplated herein.

It is nevertheless preferred to use the compound of the invention at lower dosages as the commonly used dosage of Dox. This is because lower dosages safe costs and reduce the risk of unwanted side effects, in particular on the human microbiome. For this reason even more preferred are with increasing preference dosage ranges of 1 mg per day to 75 mg per day, 1 mg per day to 50 mg per day and 1 mg per day to 25 mg per day.

Also the lower value 1 mg per day in each of the above indicated ranges may be increased and independently for each range with increasing preference to at least 2 mg per day, at least 5 mg per day, at least 7.5 mg per day and at least 10 mg per day.

These dosages per subject per day are particularly suitable for oral administration. A typical dosage regimen for oral administration is one dosage in the ranges as indicated above every 12 hours on day 1 followed by the same dose daily in 1 or 2 divided doses. A typical dosage regimen for intravenous or administration is one dosage in the ranges as indicated above on day 1 in 1 or 2 infusions followed by the same dosage or half of that daily.

In accordance with a further preferred embodiment of the invention the compound displays a minimum inhibitory concentration (MIC) for *E. coli* of at least 8 µg/mL and/or for *P*. *aeruginosa* of at least 4 µg/mL

The MIC for *E*. *coli* is preferably at least 16 µg/mL, and more preferably at least 24 µg/mL. The MIC for *P. aeruginosa* is preferably at least 25 µg/mL, and more preferably at least 50 µg/mL.

The minimum inhibitory concentration (MIC) is defined as the lowest concentration of an antimicrobial that inhibits the visible growth of a microorganism after overnight incubation. MICs are used by diagnostic laboratories to confirm resistance and also as a research tool to determine the *in vitro* activity of antimicrobials.

The procedure of a MIC test is well established in the art and may, for example, comprise: (i) A pure culture of a single microorganism (e.g. a bacterium) is grown in a suitable medium or broth, such as Mueller-Hinton broth. (ii) The culture is standardized using standard microbiological techniques to have a concentration of very near 1 million cells per milliliter. The more standard the microbial culture, the more reproducible the test results. (iii) The antimicrobial agent is diluted a number of times, usually 1:1, through a sterile diluent (typically a suitable medium or broth, such as Mueller-Hinton broth). After the antimicrobial agent has been diluted, a volume of the standardized inoculum equal to the volume of the diluted antimicrobial agent is added to each dilution vessel, bringing the microbial concentration to approximately 500,000 cells per milliliter. (iv) The inoculated, serially diluted antimicrobial agent is incubated at an appropriate temperature for the test organism for a pre-set period, usually 12 to 24 hours, such as about 18 hours. (vi) After incubation, the series of dilution vessels is observed for microbial growth, usually indicated by turbidity and/or a pellet of microorganisms in the bottom of the vessel. The last tube in the dilution series that does not demonstrate growth corresponds with the minimum inhibitory concentration (MIC) of the antimicrobial agent.

It is shown in the examples herein below that the preferred compounds of the invention 9-TB, 7,9-bis3POyr26 diF dox**,** as well as the reference compounds 7-bromo sancycline and 8-bromo sancycline display a significantly higher MIC for *E. coli* and *P. aeruginosa* than Dox. This is particularly advantageous for the medical use of the present invention which relates to the treatment or prevention of a respiratory viral disease or viral infection and not the use of the compounds of the invention as an antibiotic.

For this reason compounds that do not have a significant or a minimized antibiotic activity are advantageous in the context of the present since they do not unnecessarily distort the microbial balance of the body, in particular the intestinal flora while still having the desired effect on the respiratory viral disease or infection.

In accordance with a yet further preferred embodiment of the invention the compound is capable of activating an adaptive mitochondrial stress response.

Cells constantly monitor the function of their mitochondria and activate adaptive mitochondrial stress responses (MSR) to maintain or restore mitochondrial homeostasis upon stress. Mitohormesis is the phenomenon that ensues when these adaptive responses surpass the initial stress and lead to overall beneficial consequences on cellular and organismal fitness.

It is demonstrated in the examples herein below that the tetracycline derivatives according to the invention induce a mild adaptive mitochondrial stress response (MSR), which involves both the ATF4-mediated integrative stress response and type I interferon (IFN) signaling and also induces mitohormesis.

The mitochondrial stress response activated by a potentially damaging stimulus requires a coordinated dialogue with the cellular nucleus, known as mitonuclear communication. This interplay induced by the hormetic response in mitochondria relies on a variety of signals among which the most relevant ones are reactive oxygen species (ROS), mitochondrial metabolites, proteotoxic signals, the mitochondria-cytosol stress response, and the release of mitokines. The activation of the mitohormetic response increases lifespan in different animal models, from worms to mammals. Further, mitohormesis also enhances healthspan, particularly improving metabolism and immune system. Although multiple mediators and stress signals have been proposed to activate this protective mechanism, beneficial outcomes of mitohormesis are most probably due to a moderate increase in mitochondrial ROS (reviewed in Barcena et al. Int Rev Cell Mol Biol, 2018; 340:35-77) or due to lowering of mitochondrial preoteotoxic stress (reviewed in Mottis et al. Science, 2019: 366: 827-832).

Several techniques for determining whether a compound is capable of activating an adaptive mitochondrial stress response are known in the art and are also illustrated by the examples.

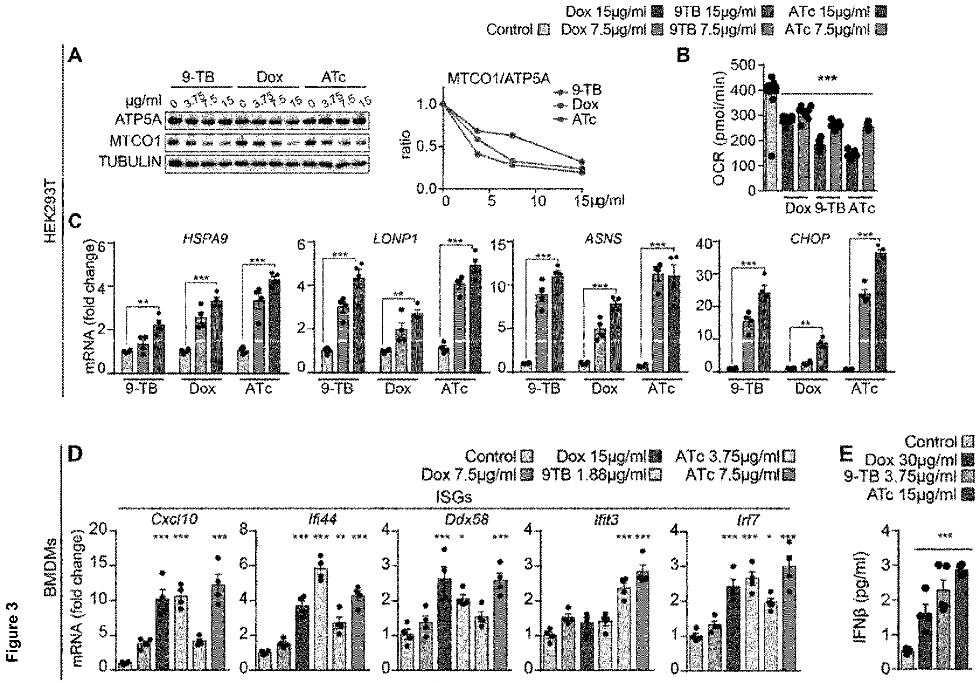

For instance, MSR may be measured by measuring the mitonuclear protein imbalance. The mitonuclear protein imbalance may be determined by a disbalanced ratio between a mitochondrial-encoded gene (e.g. MTCO1) versus a nuclear-encoded gene (e.g. ATP5A) of OXPHOS subunits as is illustrated by Figure 3A.

MSR may also be measured by measuring reduced basal oxygen consumption rate (OCR) in cells, such as HEK393T cells, preferably in a dose-dependent manner as illustrated by Figure 3B. A further possibility is the measurement of IFNβ release in cells, preferably murine bone marrow-derived macrophages (BMDMs). In this connection it is preferred that an at least 25% reduction of OCR in HEK393T cells and/or an at least 2-fold increase in IFNβ release in murine BMDMs indicates a compound being capable of activating an adaptive mitochondrial stress response.

Yet further, MSR may also be measured by determining the induction of transcripts for the mammalian MSR signature genes (e.g. at least two, three, four or all genes of Cxcl10, Ifi44, Ddx58, Ifit3 and Irf7). The induction of transcripts can be determined, for example, by RT-qPCR, wherein an increase of the transcripts of >20%, preferably >50%, and most preferably above 100% as comrade to the absence of the compound according to the invention indicates the activating of an adaptive MSR.

A prototypical and well-studied form of the MSR is the mitochondrial unfolded protein response (UPRmt), first described in mammalian cells (1), but more extensively characterized in *Caenorhabditis elegans* (*C. elegans*) (reviewed in (2-4)).

MSR is preferably measured herein by measuring UPRmt induction. UPRmt induction is in turn preferably measured by using a *C. elegans* hsp-6::gfp reporter strain with Dox administration and cco-1 RNAi feeding as positive controls, as previously described (25) and in Figure 2. In this model, worm strains expressing a GFP reporter fused to promotors of HSP- and HSP-60 proteins, are used to evaluate the activation of the UPRmt, where proteotoxic stress can be induced by small molecule compounds and effectors. In one such activator the compound is paraquat, which can induce excessive production of reactive oxygen species leading to cellular dysfunction and apoptosis, while changes in OXPHOS components and their stoichiometric balance can induce UPRmt. In other instances, UPRmt originates in the mitochondrial matrix, where malformed proteins overload the chaperone pool, cleaving proteins into peptides that can decrease transporter importation of the leucine zipper translocation factor ATFS-1, a translocator of ATP and other metabolites and a major regulatory protein capable of binding to the nuclear localization export protein and mitochondrial targeting sequences linking the nucleus and the mitochondria. Once in the nucleus ATFS-1 induces mitochondrial quality control by activation of genes controlling proteases, chaperones, proteins involved in mitochondrial translocation, ROS detoxification, and protection from mitochondrial dysfunction. ATFS-1 also regulates cell metabolism, shifting from respiration to glycolysis for ATP generation during mitochondrial stress. Further details on measuring UPRmt induction can be found in the sections "Compound screening in C. elegans" and "C. elegans experiments in microfluidic system" herein below in Example 3.

The compound is preferably capable of activating an adaptive MSR which increasing preference at least 1.5, at least 2 and at least 5 stronger than the adaptive MSR of Dox and/or Minocycline as positive control in the same MSR measurement.

In the case of measuring UPRmt induction the read-out of the measurement is preferably the GFP fluorescence intensity. Hence, in this case the GFP fluorescence intensity of the compound according to the invention is at least at least 1.5, at least 2 and at least 5 times stronger than the GFP fluorescence intensity as compared to Dox and/or Minocycline as positive control.

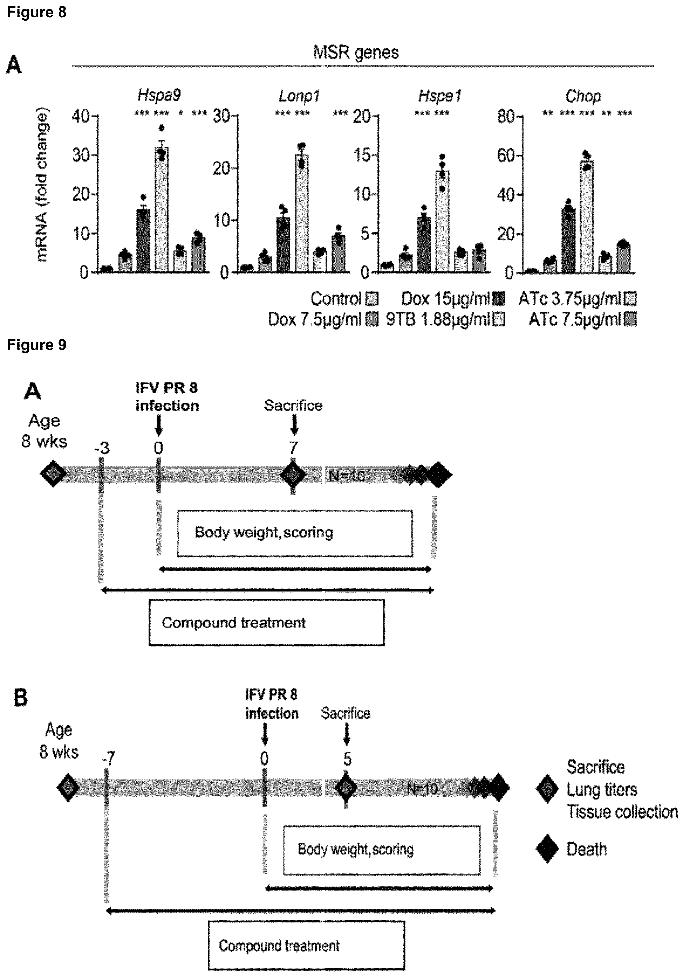

The Figures show.

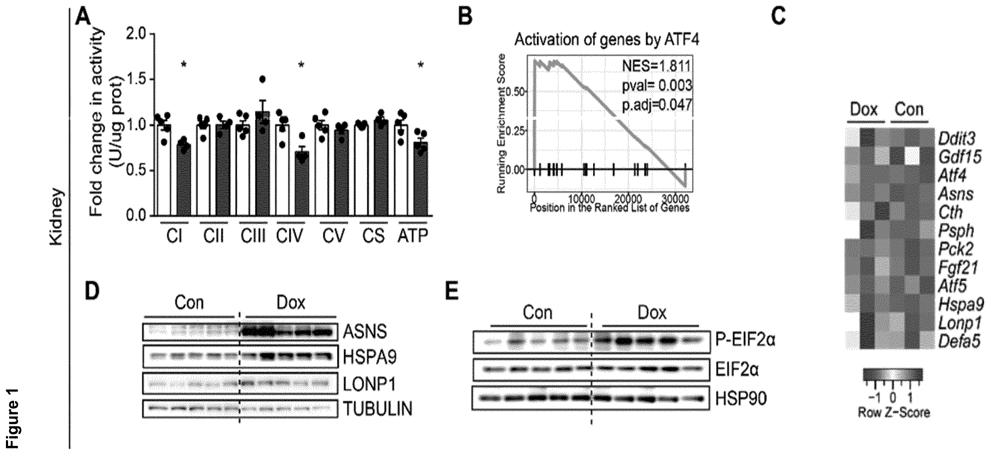

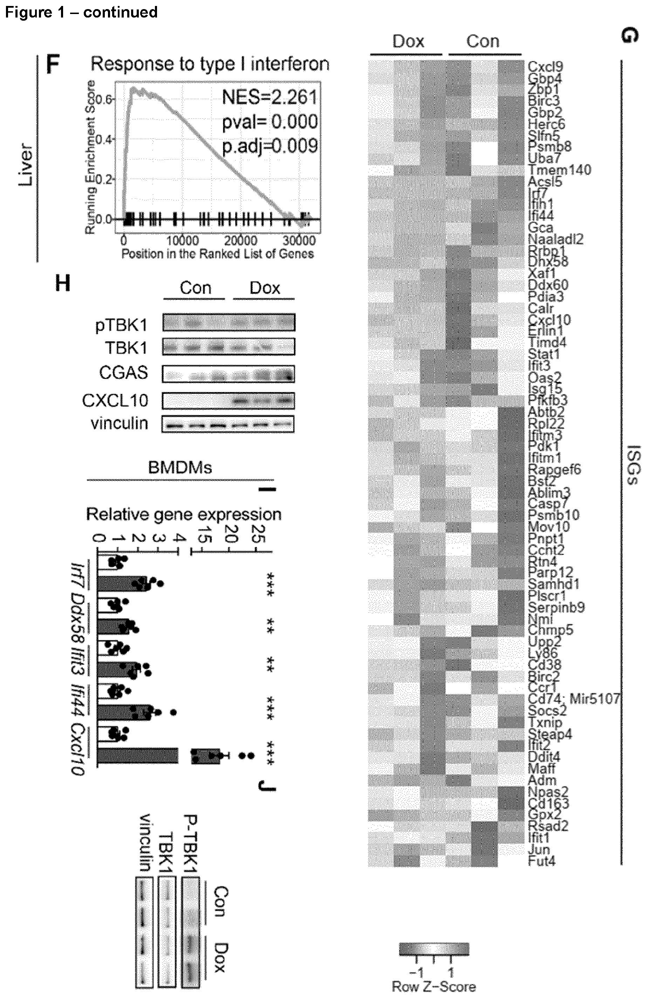

**Figure 1****: Doxycycline induces the ATF4 response and the type** I IFN **response.**
   A. Biochemical measurement of oxidative phosphorylation (OXPHOS) complexes and ATP levels in the kidney of germ-free C57BL/6J male mice raised and maintained in a germ-free environment and that were drinking regular water or water supplemented with doxycycline (Dox) at 500 mpkd for 16 days (n=5). B-C. Enrichment score plot for the gene set "Reactome Activation of genes by ATF4" (B) and heatmap representing the transcript levels of ATF4/5 targets (C) from kidney transcriptomics data of control vs Dox-treated germ-free mice. D. Western blot analysis of selected ATF4 targets in the kidneys of germ-free mice. E. Immunoblots of phosphorylated EIF2α (P-EIF2α) and total EIF2α (E) in kidneys of Dox-treated germ-free mice. F-G. Enrichment score plot for the GO term "Response to type I interferon" (F) and heatmap representing the transcript levels of some interferon-stimulated genes (ISGs) (G) from livers of germ-free mice treated with Dox. **H.** Immunoblots of phosphorylated TBK1 (pTBK1), TBK1, the ISG proteins, CGAS and CXCL10, and VINCULIN as loading control. **I.** Transcript levels of selected ISGs of bone marrow-derived macrophages (BMDM) (day 6 of differentiation, derived from C57BL/6J mice) treated with Dox at 30µg/ml for 9 hours (n=6). **J.** Immunoblots of phosphorylated TBK1 (pTBK1), TBK1, and vinculin as control in BMDM treated with Dox at 30µg/ml for 3 hours. **K.** Amplification of different mtDNA regions by qPCR in the cytosolic fraction of BMDMs with Dox at 30µg/ml for 1 hour (n=10). **L.** Levels of interferon β (IFNβ) in the culture medium of BMDM treated with Dox (30µg/ml for 14 hours) and/or 2',3' dideoxycytidine (ddC at 100µM for 72 hours) (n=8). A, I, K: Statistical analysis was performed by Student t-test p-value **p*≤0.05; ***p*≤0.01; ****p*≤ 0.001. L: Statistical analysis was performed by one-way ANOVA followed by Tukey post-hoc correction (**p* ≤ 0.05, ***p* ≤ 0.01, ****p* ≤ 0.001). All: Error bars represent standard error mean (SEM).
**Figure 2****: Selecting Tet derivatives that induce UPR^{mt} in *C. elegans.***
   **A.** Structural locants of the Tet scaffold and UPR^{mt}-active and -inactive compounds by chemically modified positions (based on activity of the *hsp-6::gfp* reporter, Fig. 2D). **B.** Minimum Inhibitory Concentrations (µg/mL) of bacterial growth for indicated Tet derivatives and bacterial strains. **C.** Chemical structures of the Tet derivatives shown in (A & B). **D.** Representative images of the induction of the UPR^{mt} in the *C. elegans hsp-6::gfp* reporter strain (25) exposed to the indicated tetracycline derivatives at 68 µM (except for 9-TB, which is at 17 µM) since the parental L4 stage. Dox and OXPHOS LOF through feeding *cco-1* RNAi serve as positive controls. The pictures show the progeny at day 2-3 of adulthood (similar exposure time for all images; GFP-fluorescence in top raw, differential interference contrast (DIC) in bottom raw). **E.** Dose-response for the UPR^{mt} activation (*hsp-6::gfp* reporter strain) upon exposure to different concentrations of Dox, 9-TB, ATc or treatment with *cco-1* RNAi using an automated microfluidic device (33) (n=14-16). Statistical analysis was performed by one-way ANOVA followed by Bonferroni post-hoc correction (**p* ≤ 0.05, ***p* ≤ 0.01, ****p* ≤ 0.001). Error bars represent standard deviation (SD).
**Figure 3****: Tet derivatives induce the MSR and type** I IFN **signalling in mammalian cells.**
   **A-C.** Tet derivatives induce a mito-nuclear protein imbalance and the MSR in human HEK293T cells treated for 24 hours at the indicated concentrations. **A.** Immunoblots of HEK293T cells for the OXPHOS subunits ATP5A (encoded in nuclear DNA) and MTCO1 (encoded in mitochondrial DNA) with TUBULIN serving as a control. Quantification of the relative MTCO1/ATP5A ratio is shown on the right. **B.** Oxygen consumption rate of HEK293T cells exposed to different concentrations of Dox, 9-TB, or ATc (n=8). **C.** Transcript levels of the indicated MSR genes measured by RT-qPCR (n=4). **D-E.** Tet derivatives induce transcript levels of the indicated ISGs (D) and stimulate IFNβ secretion (E) after 24 hours treatment at the indicated concentrations in mouse BMDM (day 6 differentiation) (n=4). Statistical analysis was performed by ANOVA followed by Tukey post-hoc test (**p* ≤ 0.05,***p* ≤ 0.01, ****p* ≤ 0.001). Error bars represent standard error mean (SEM).

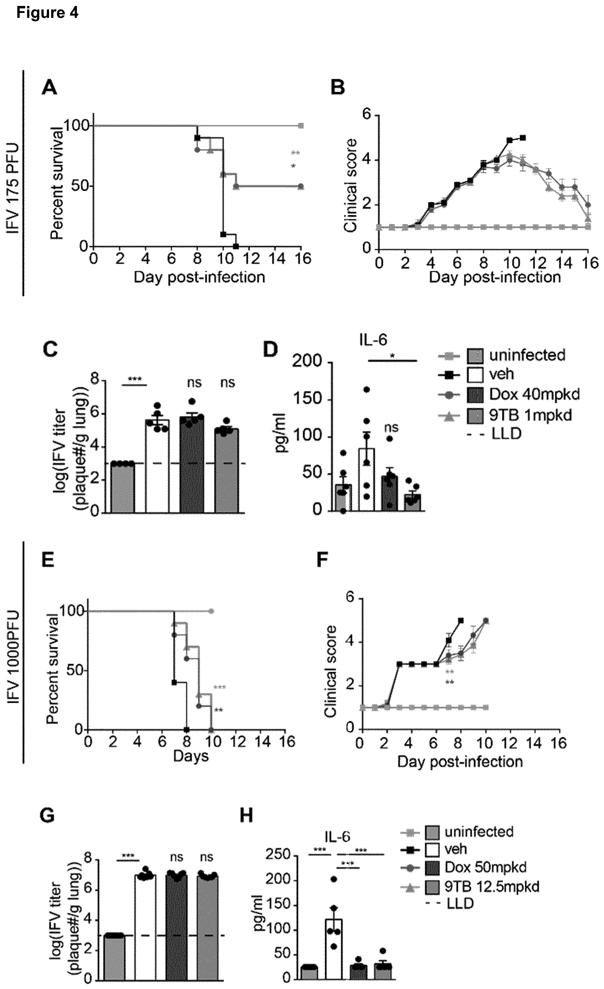

**Figure 4****: Tets mediate disease tolerance to IFV in mice.**
   **A-D.** 8-weeks old BALB/cN mice (n=15-16) were injected with Dox (40 mpkd) or 9-TB (1 mpkd) and intranasally infected with 175 PFU of IFV-A H1N1 PR8, as described in Fig. 9A. Survival (A) and clinical score (B) were followed for 16 days post-infection (n=10). At day 7 post-infection, viral titers in lung lysates (C, n=5) and IL-6 levels in plasma (D, n=6) were measured (n=5). **E-H.** 8-weeks old BALB/cN (n=15-16) mice were injected with Dox (50 mpkd) or 9-TB (12.5 mpkd) and intrasanally infected with 1000 PFU of IFV-A H1N1 PR8, as shown in Fig. 9B. Survival (E) and clinical score (F) were followed over 10 days post-infection (n=10). At day 5 post-infection, viral titers in lung lysates (G) and IL-6 levels in plasma (H) were measured (n=5). Statistical analysis was performed by ANOVA followed by Tukey post-hoc test (**p*≤0.05,***p* ≤ 0.01, ******p* ≤ 0.001). For survival curves, statistical analysis was performed by Log-rank (Mantel-Cox) test (**p* ≤ 0.05,***p* ≤ 0.01, ****p* ≤ 0.001). Error bars represent standard error mean (SEM).

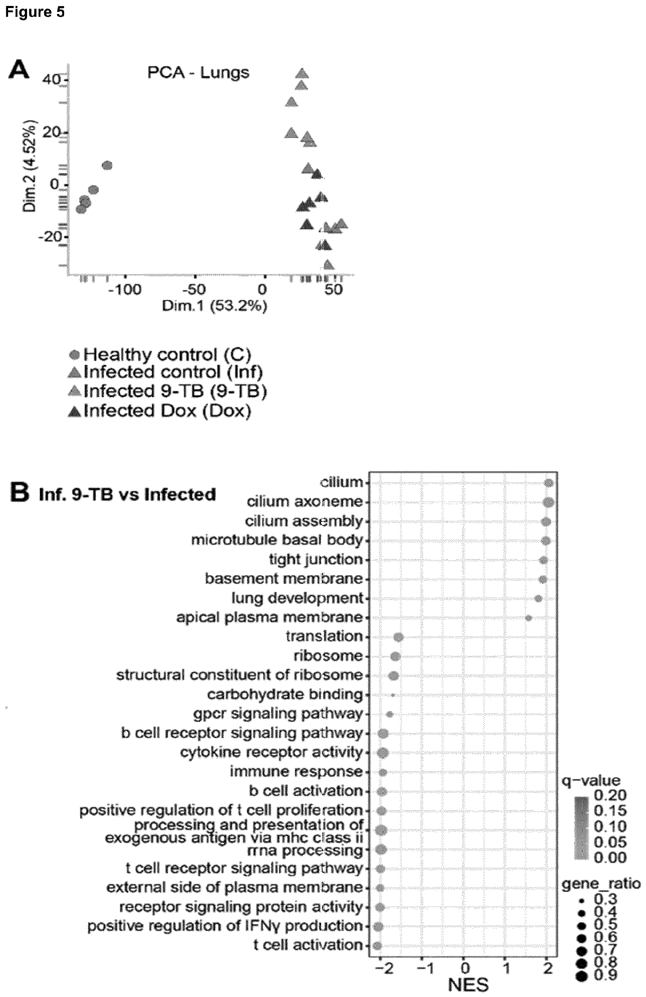

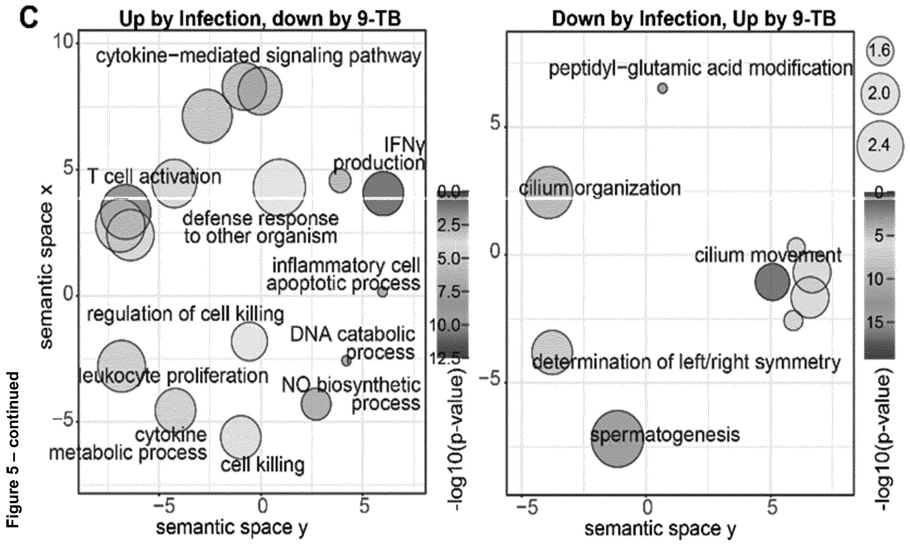

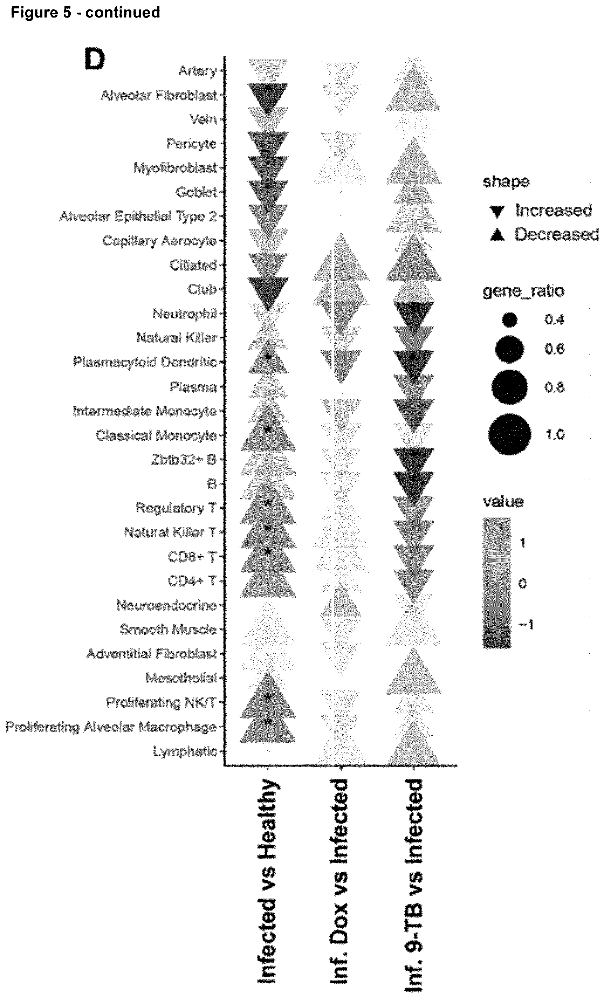

**Figure 5****: 9-TB counteracts the inflammatory and lung damaging effects of IFV infection.**
   **A.** Principal component analysis (PCA) of lung RNAseq transcriptomes collected at day 7 post-infection of BALB/cN mice with 175 PFU IFV-A H1N1 PR8 (n=5-6). **B.** Gene set enrichment analysis (GSEA) results for Gene Ontology (GO) gene sets modulated in the comparison between 9-TB treated versus control IFV-infected mice. A positive, respectively negative, normalized enrichment score (NES) corresponds to an overall up-regulation, respectively down-regulation, of the corresponding gene set. **C.** Revigo analysis summarizing the main themes in the significantly enriched GO Biological Process (GOBP) sets amongst genes induced by IFV infection and down-regulated by 9-TB (left panel), and genes down-regulated by IFV infection and induced by 9-TB (right panel). **D.** GSEA results of the RNA-Seq data showing the directionality (increase or decrease) of the modulated lung cell transcript profiles based on common markers shared by both human and mouse lung cell types derived from extant single cell transcriptomic data (43).

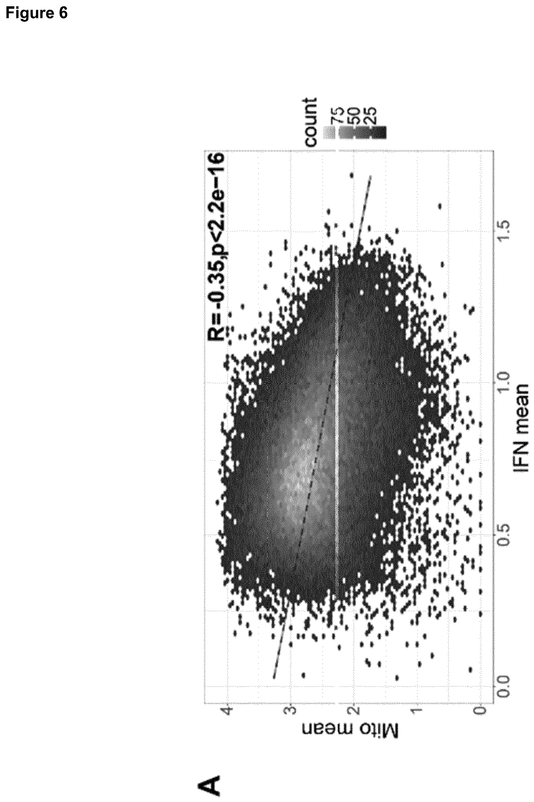

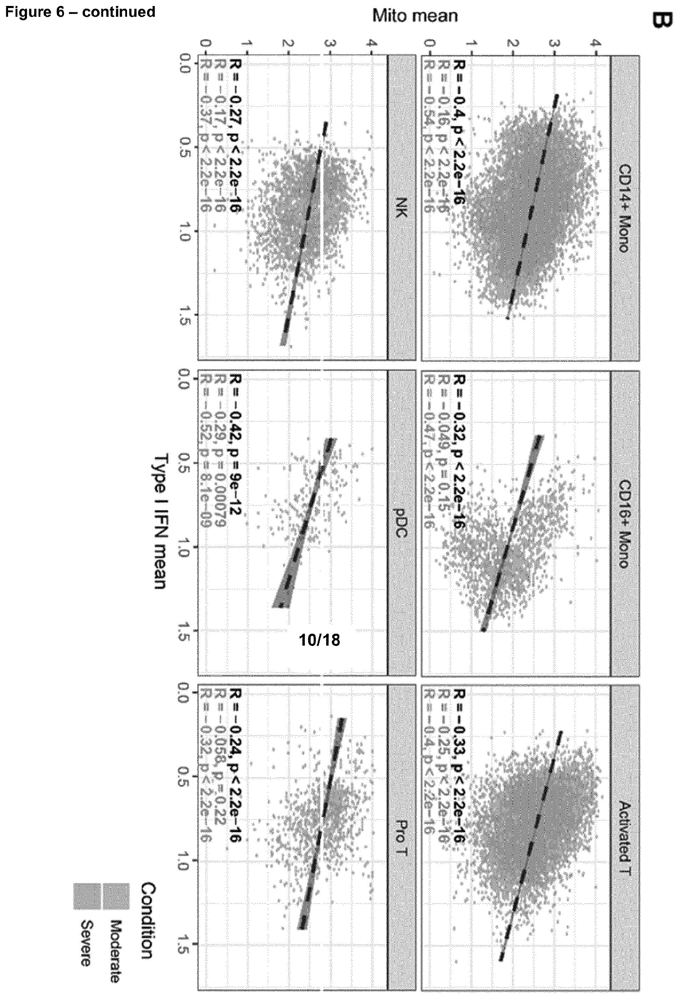

**Figure 6****: Inverse correlation between mitochondria-encoded and type** I IFN **genes in PBMCs of COVID-19 patients.**
   **A.** Density scatterplot relating the mean expression of the Type I IFN GO term genes (GO:0034340) ("IFN_mean", x axis) and the mean expression of the mitochondria-encoded genes ("Mito_mean", y axis) for the whole of all single-cell data points measured in peripheral blood mononuclear cells (PBMCs) isolated from COVID-19 patients in (38). The color of each hexagonal dot represents the plotting density, meaning that a yellow dot represents 100 single-cell data points. The line represents the Pearson correlation and the associated coefficients are indicated. **B.** Scatterplot relating the mean expression of the Type I IFN GO term genes (GO:0034340) ("IFN_mean", x axis) and the mean expression of the mitochondria-encoded genes ("Mito_mean", y axis) in single-cell RNA-Seq data of the indicated cell types measured in PBMCs isolated from COVID-19 patients. The distribution of single-cell data points is modelized by hexagonal 2D bin plot. Orange and light blue correspond respectively to the distribution of single-cell data points of respectively moderate and severe COVID-19 cases as defined in (38), darker blue corresponding to the overlap of orange and blue dots. Mono= monocytes, NK= natural killer, pDC=plasmacytoid dendritic cells.

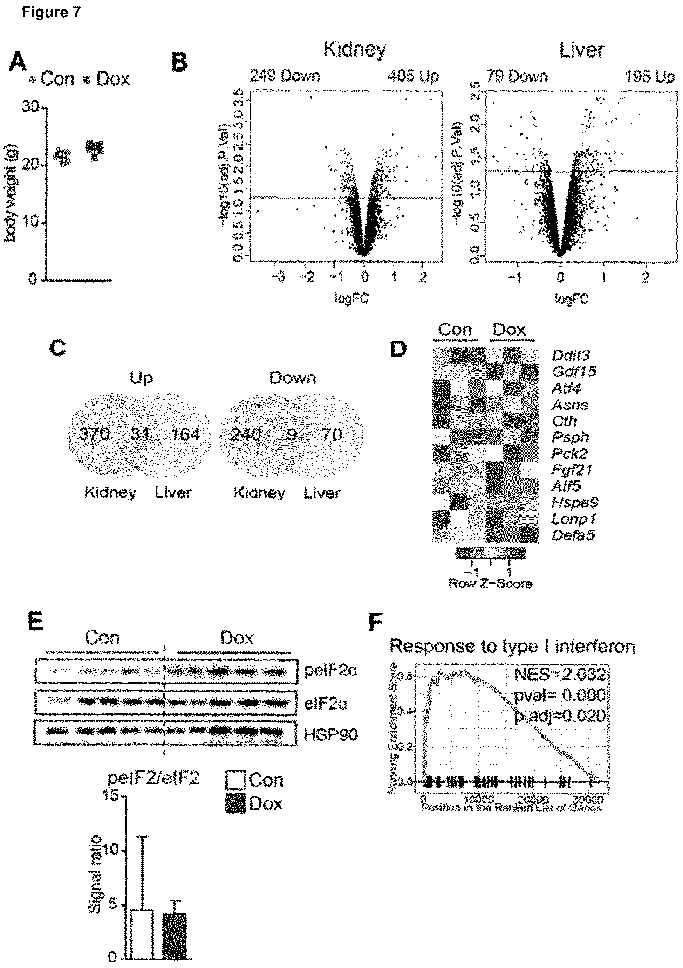

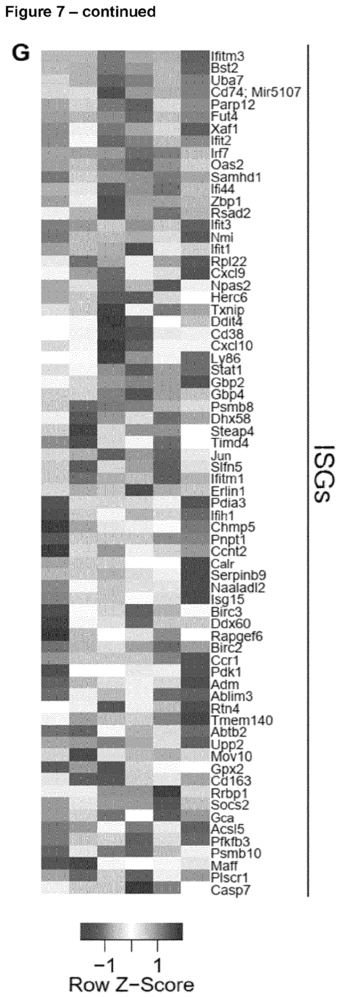

**Figure 7****: Doxycycline induces the ATF4 and the type** I IFN **response.**
   **A.** Body weight at the time of sacrifice of germ-free C57BL/6J male mice treated with Dox at 500mpkd. **B.** Volcano plots displaying -log(adj. p-value) and the log fold change (FC) in expression of genes in kidney and liver. Above the graph, the number of differentially expressed genes is indicated (genes with adjusted p value (adj.pval)<0.05). **C.** Venn diagrams displaying the genes commonly up and down-regulated in liver and kidney of germ-free mice treated with Dox at 500mpkd (differentially expressed genes with adj.pval<0.05). **D.** Heatmap representing the transcript levels of ATF4/5 target genes from liver transcriptomics data of control vs Dox-treated germ-free mice. **E.** Immunoblots of phosphorylated eIF2α (peIF2α) and total eIF2α and quantification in liver of Dox-treated germ-free mice. **F-G.** Enrichment score plot for the GO term "Response to type I interferon" (F) and heatmap representing the transcript levels of some interferon-stimulated genes (ISGs) (G) from kidneys of germ-free mice treated with Dox.
**Figure 8****: Tet derivatives induce MSR genes in mammalian cells.**
   **A.** Transcripts levels of the indicated MSR genes measured by RT-qPCR after 24-hours treatment at the indicated concentrations of the Tet derivatives in mouse bone marrow derived macrophages (day 6 differentiation). Statistical analysis was performed by ANOVA followed by Tukey post-hoc test (**p*≤0.05,***p* ≤ 0.01, ****p* ≤ 0.001). Error bars represent standard error mean (SEM).

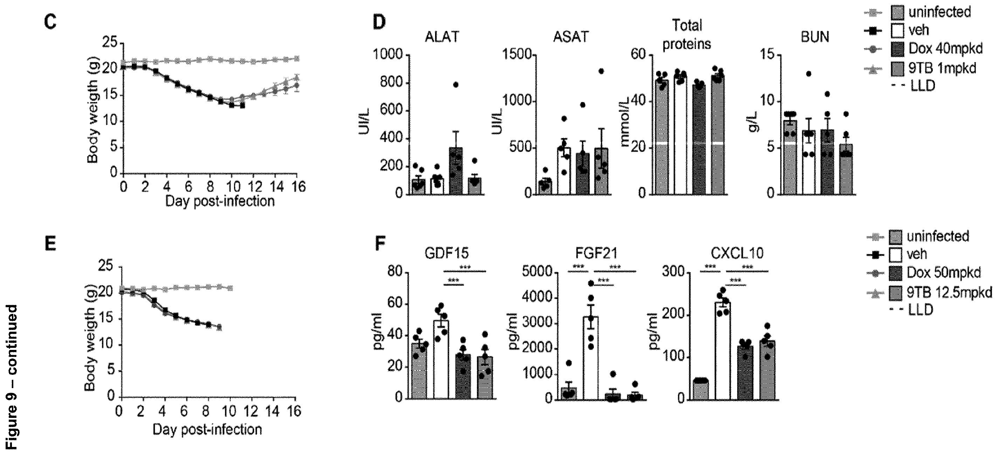

**Figure 9****: Tets mediate disease tolerance to IFV in mice.**
   **A-B.** Descriptive schemes of the 2 respective mouse IFV infection experiments. **C-D.** 8 weeks-old BALB/cN mice (n=15-16) were injected with Dox (40 mpkd) or 9-TB (1 mpkd) and intrasanally infected with 175 PFU of IFV-A H1N1 PR8. **C.** Body weight was followed for 16 days post-inoculation (n=10). **D.** At day 7 post-infection, plasma levels of alanine aminotransferase (ALAT), asparagine aminotransferase (ASAT), total proteins and blood urea nitrogen (BUN) (n=5). **E-F.** 8 weeks-old BALB/cN (n=15-16) mice were injected with Dox (50 mpkd) or 9-TB (12.5 mpkd) and intrasanally infected with 1000 PFU of IFV-A H1N1 PR8, as described in Fig. 9B. E. Body weight was followed for 10 days post-inoculation (n=10). F. At day 5 post-infection, plasma levels of GDF15, FGF21 and CXCL10 in plasma were measured (n=5). Statistical analysis was performed by ANOVA followed by Tukey post-hoc test (**p*≤0.05,***p* ≤ 0.01, ****p* ≤ 0.001). Error bars represent standard error mean (SEM).

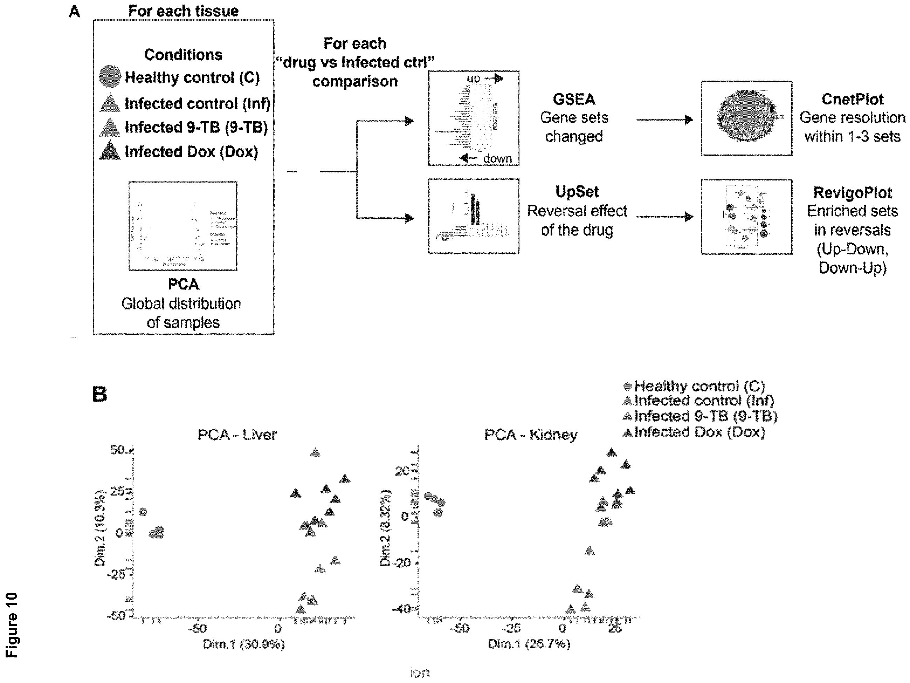

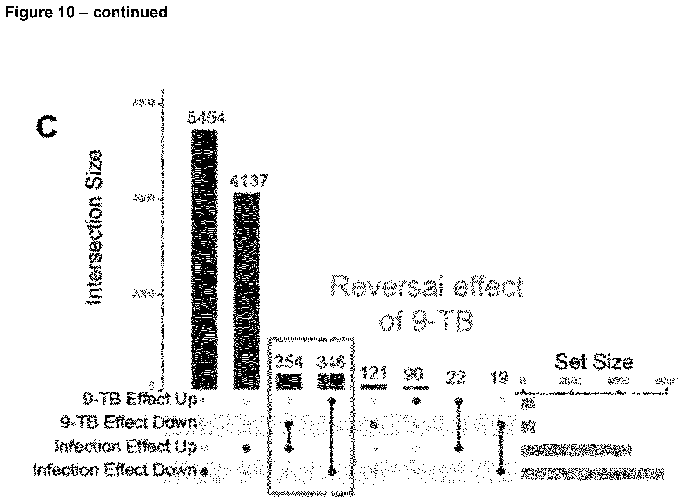

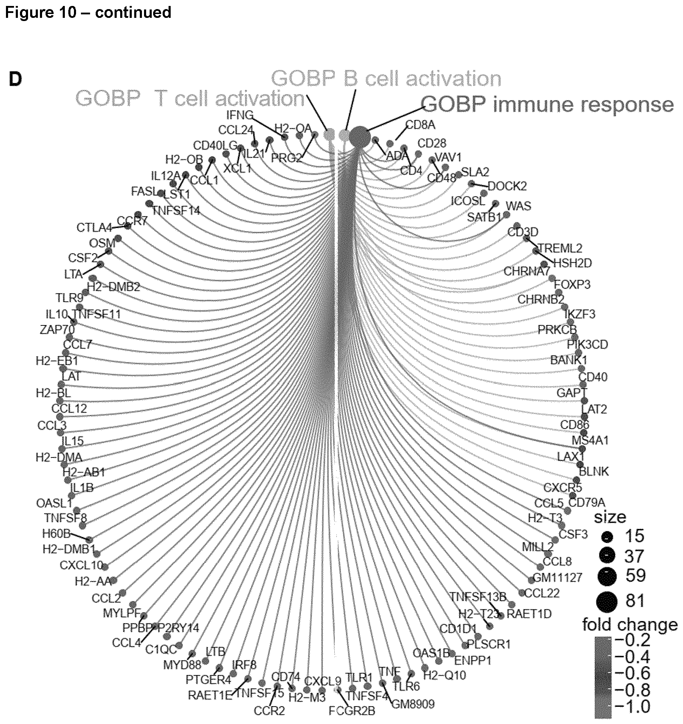

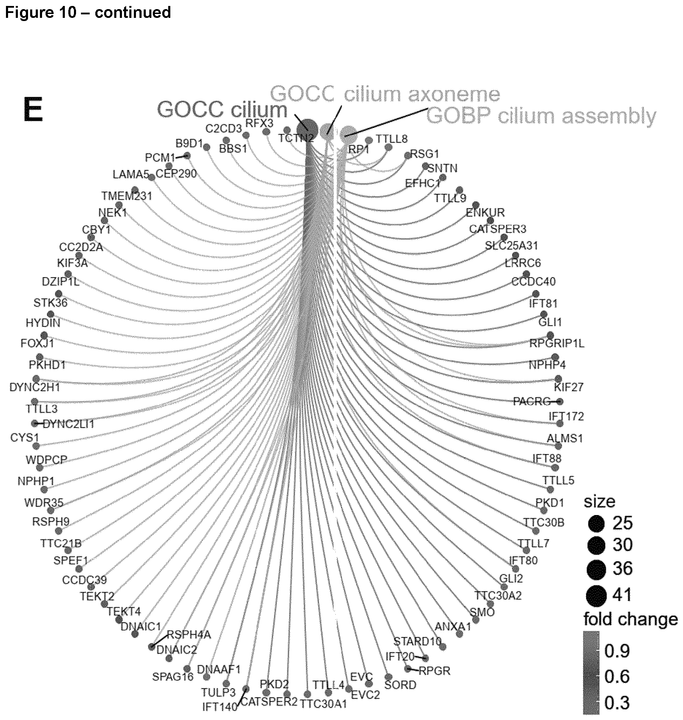

**Figure 10****: 9-TB counteracts the inflammatory and lung damaging effects of IFV infection.**
   **A.** Scheme summarizing the pipeline used for the analysis of transcriptomics data in the tissues. **B.** Principal component analysis (PCA) of liver and kidney transcriptomes collected at day 7 post-infection of BALB/cN mice with 175 PFU IFV-A H1N1 PR8 (n=5-6). **C.** UpSet R plot summarizing the effects of IFV and 9-TB on significantly changed genes in the lung RNA-Seq transcriptomics in 4 different categories (up/down-regulated by IFV/9-TB; with filtering features adj. p-value ≤ 0.05 and absolute fold change ≥ 0.5). A link between 2 dots symbolizes the intersection between the 2 corresponding categories and the corresponding bar right above symbolizes the amount of genes in this intersection. **D-E.** Cnetplots for immune-related (D) and cilium-related (E) gene sets, respectively, representing the transcript levels differentially expressed between 9-TB *versus* IFV-infected conditions.

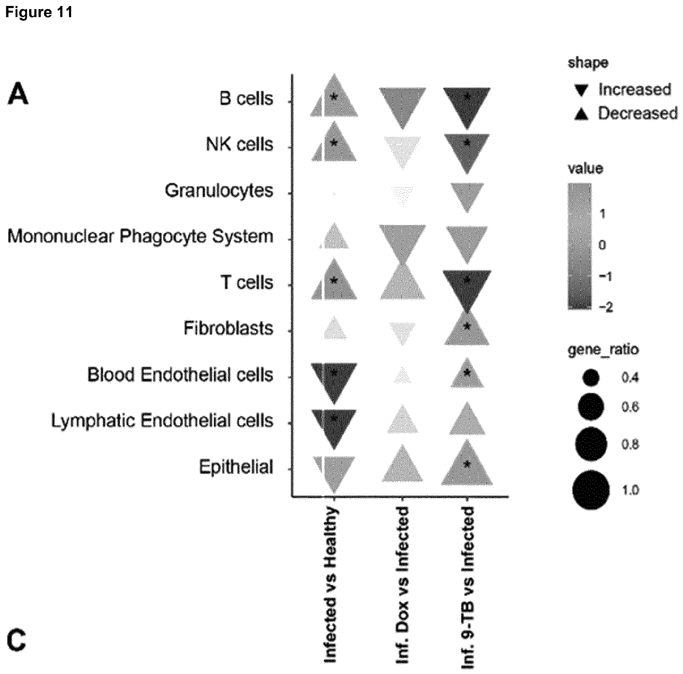

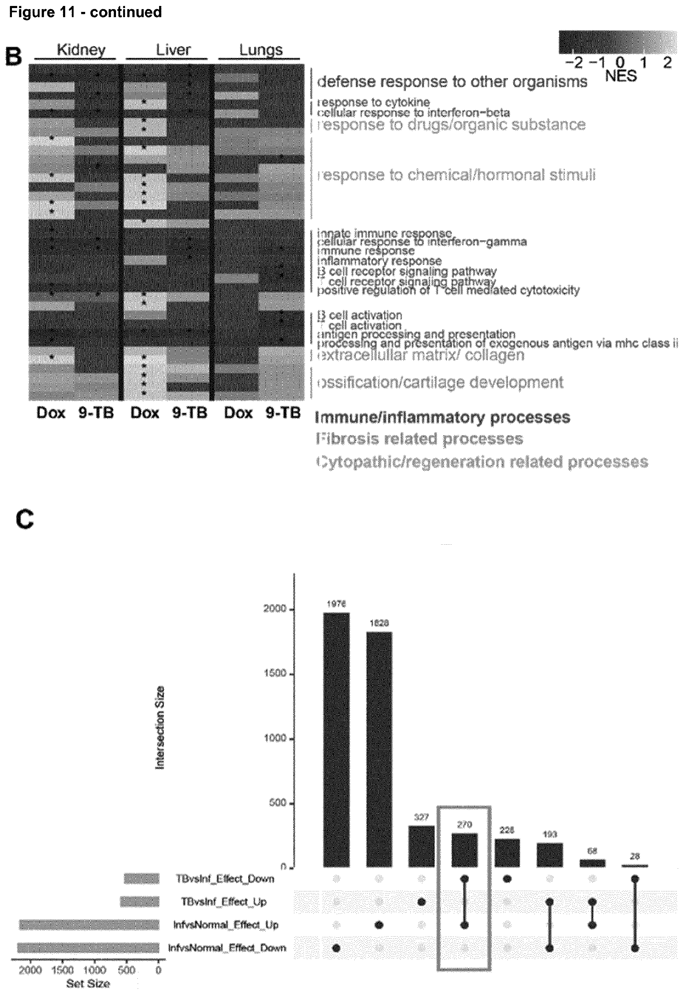

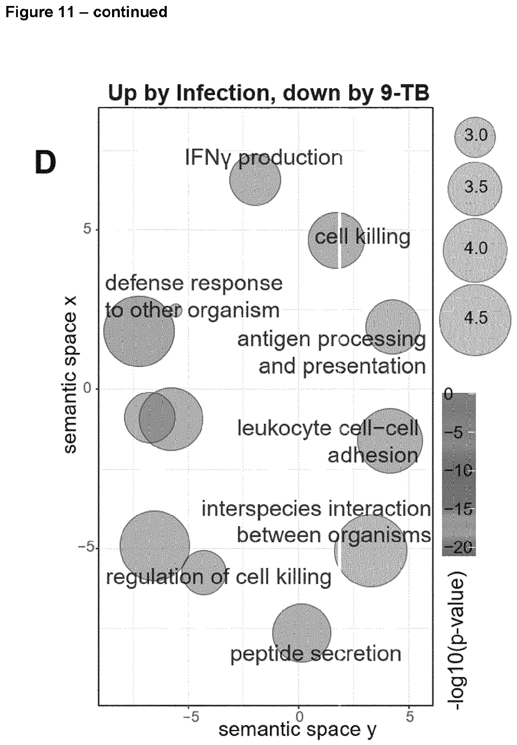

**Figure 11****: 9-TB counteracts the inflammatory and lung damaging effects of IFV infection. (continued)**
   **A.** GSEA results of the RNA-Seq data (Fig. 5) showing the directionality (increase or decrease) of the significantly modulated lung cell transcript profiles based on markers of mouse lung cell types described in (*12*). **B.** Heatmap summarizing the normalized enrichment score (NES) result of the GSEA for chosen gene sets for the effect of 9-TB and Dox compared to the IFV condition. **C.** UpSet R plot showing the effects of IFV and 9-TB on significantly changed genes in the liver RNA-Seq transcriptomics in 4 different categories (up/down-regulated by IFV/9-TB; with filtering features adj. p-value ≤ 0.05 and absolute fold change ≥ 0.5). A link between 2 dots symbolizes the intersection between the 2 corresponding categories and the corresponding bar right above symbolizes the amount of genes in this intersection. **D.** Revigo analysis summarizing the main themes in the significantly enriched GO Biological Process (GOBP) sets amongst genes induced by IFV infection and down-regulated by 9-TB (left panel), as defined in the Upset R plot (Fig. 11C).

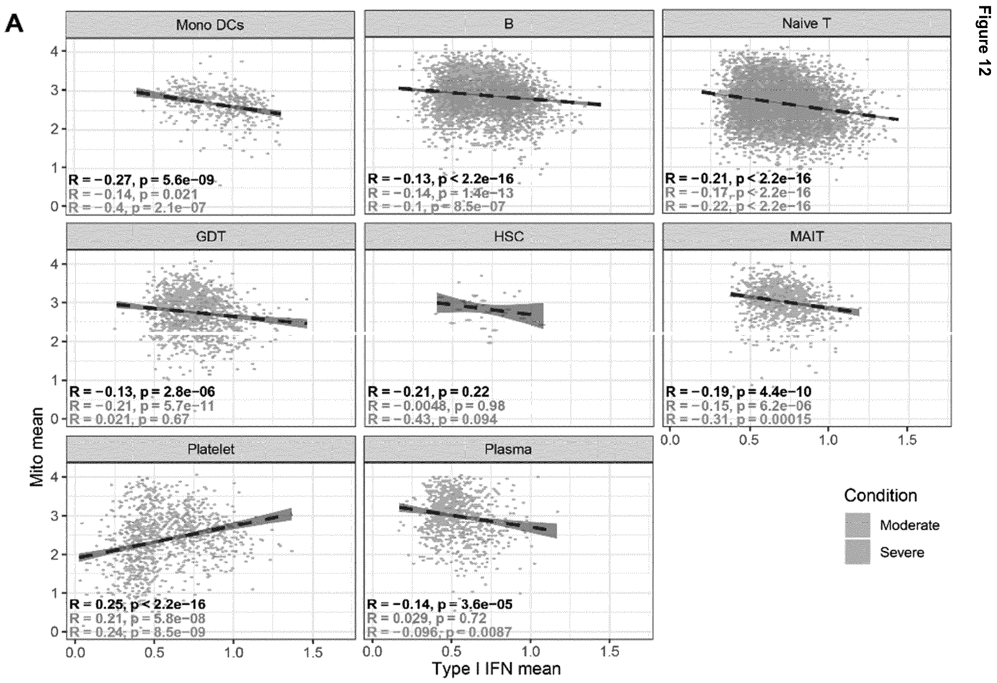

**Figure 12****: Inverse correlation between mitochondria-encoded and type** I **IFN genes in IFV-infected mice and COVID patients.**
   **A.** Scatterplot relating the mean expression of the type I IFN GO term genes (GO:0034340) ("IFN_mean", x axis) and the mean expression of the mitochondria-encoded genes ("Mito_mean", y axis) in single-cell RNA-Seq data of the indicated cell types measured in PBMCs isolated from COVID-19 patients. The distribution of single-cell data points is modelized by hexagonal 2D bin plot. Orange and light blue correspond respectively to the distribution of single-cell data points of respectively moderate and severe COVID-19 cases as defined in (13), darker blue corresponding to the overlap of orange and blue dots. GDT= γδ T cells, HSC= hematopoietic stem cells, MAIT= mucosal associated invariant T cells.

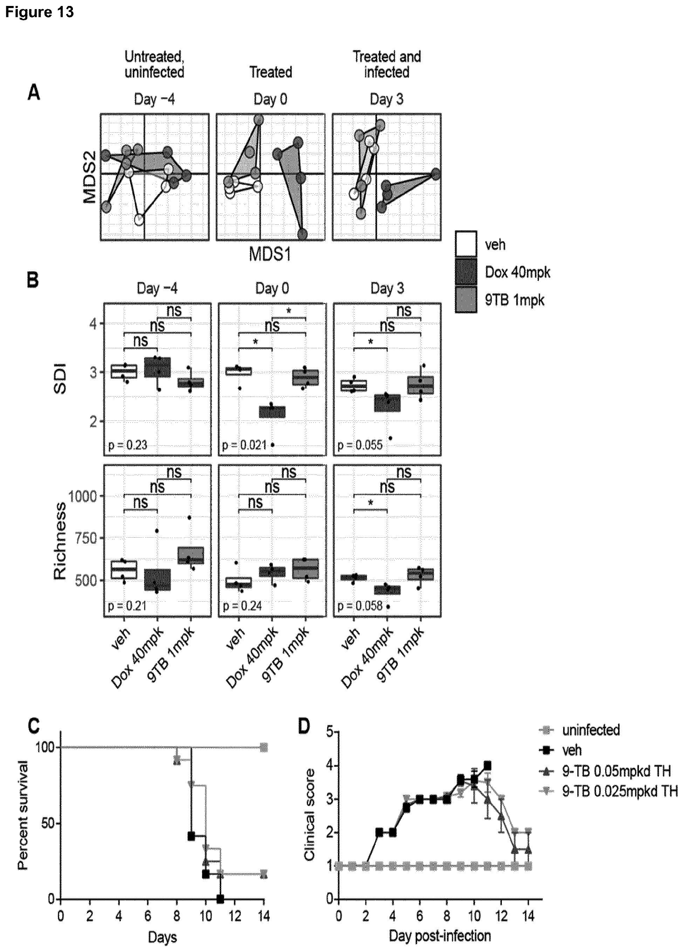

**Figure 13****:** **Figure 1****: 9-TB does not impact on gut microbiome and shows encouraging effects when therapeutically administered.**
   **A.** Comparison of bacterial community composition by nonmetric multidimensional scaling (NMDS) based on the Bray-Curtis dissimilarity. **B.** Comparison of bacterial species diversity in terms of Shannon diversity index (SDI) and richness. Statistical significance assessed by Kruskal-Wallis test and post-hoc Wilcoxon test with p-value adjusted for multiple comparison using the Holm-Bonferroni method. '****': p < 0.0001, '***': p < 0.001, '**': p < 0.01, '*': p < 0.05, 'ns': p > 0.05. C-D. 8-weeks old BALB/cN mice (n=12) were infected intranasally with 760 PFU of IFV-A H1N1 PR8 and injected with 9-TB (0.05, 0.025 mpkd). Survival **(C)** and clinical score **(D)** were followed for 14 days post-infection (n=12). Statistical analysis was performed by ANOVA followed by Tukey post-hoc test (**p*≤0.05,***p* ≤ 0.01, ****p* ≤ 0.001). For survival curves, statistical analysis was performed by Log-rank (Mantel-Cox) test (**p* ≤ 0.05,**p ≤ 0.01, ****p* ≤ 0.001). Error bars represent standard error mean (SEM).

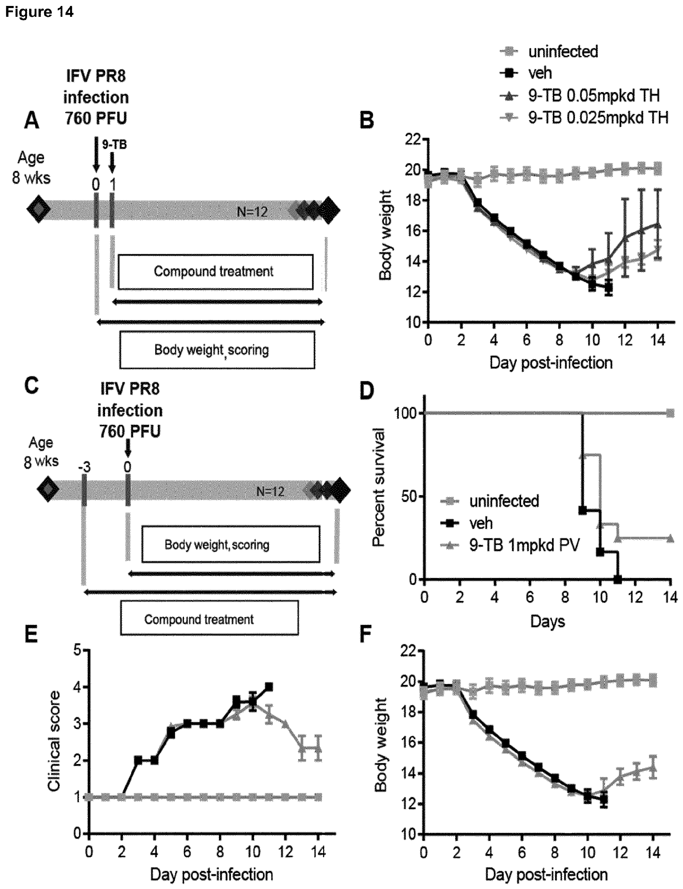

**Figure 14****: Tets mediate disease tolerance to IFV in mice.**
   **A.** Descriptive scheme of the mouse IFV infection experiment depicted in Fig. 5C-D, S5B. **B.** 8-weeks old BALB/cN mice (n=12) were infected intranasally with 760 PFU of IFV-A H1N1 PR8 and injected with 9-TB (0.05, 0.025 mpkd). Body weight was followed for 14 days post-inoculation (n=12). **C.** Descriptive scheme of the mouse IFV infection experiment depicted in Fig.S5D-F. D-F. 8 weeks-old BALB/cN mice (n=15-16) were injected with 9-TB (1 mpkd) and intranasally infected with 760 PFU of IFV-A H1N1 PR8. Survival **(D),** clinical score **(E)** and body weight **(F)** were followed for 14 days post-infection (n=12). For survival curves, statistical analysis was performed by Log-rank (Mantel-Cox) test (*p ≤ 0.05,**p ≤ 0.01, ****p* ≤ 0.001).

The examples illustrate the invention.

### Example 1 - Results

To characterize the MSR induced by Tets *in vivo,* doxycycline (Dox) was administered at 500 mg/kg/day (mpkd) in the drinking water to 9 weeks-old germ-free C57BL/6J mice for 16 days (*5*, *7*), hence eliminating the potential confounding impacts of Dox on the microbiome. Body weight at the time of the sacrifice was not different between the control and Dox-treated animals (Fig. 7A), indicating the absence of obvious adverse effects. As reported in livers of mice maintained under conventional conditions (*9*), OXPHOS complex activity as well as ATP levels were also reduced by Dox in kidneys of germ-free mice (Fig. 1A). Dox elicited organ-specific transcriptional responses, with the expression of remarkably more genes and different being affected in the kidney compared to the liver (Fig. 7B-C). In Dox-treated kidneys, gene set enrichment analysis (GSEA) (*20*) revealed the induction of the ATF4-mediated integrated stress response (ISR) (Fig. 1B), a common hallmark of the mammalian MSR (21). The MSR features the induction of mitochondrial chaperones and proteases, such as *Hspa9* and *Lonp1,* as well as of enzymes mediating adaptation to nutrient deprivation, such as asparagine synthetase (ASNS), which were increased at both the transcript and protein levels (Fig. 1C-D). In line with the activation of the ATF4-ISR pathway, the kidney displayed increased eIF2α phosphorylation (Fig. 1E), which slows down cytosolic cap-dependent translation as a compensation for energy deprivation caused by mitochondrial stress and favors the translation of ATF4 transcripts by cap-independent mechanisms (22). Dox-treated germ-free mice kidneys thus gathered the typical attributes of the ATF4-ISR, a hallmark of the mammalian response to mitochondrial stress.

In the liver, eIF2α phosphorylation and the ATF4-ISR program were not induced (Fig. 7D-E). The liver transcriptome, however, indicated that Dox induced the type I IFN response (Fig. 1F-G), which was confirmed at the protein level by the increased expression of two interferon-stimulated genes (ISGs), Cyclic AMP-GMP synthetase (CGAS) and C-X-C motif chemokine ligand 10 (CXCL10), and the increased phosphorylation of TANK-binding kinase 1 (TBK1) (Fig. 1H). The type I IFN response is an innate immune pathway that, upon sensing viral DNAs, activates cGAS-STING-TBK1 signaling, culminating in the secretion of the type I IFNs, IFNα and IFNβ, and the induction of the expression of ISGs (23). Of note, the type **I IFN** response was also induced in kidneys but to a lesser extent (Fig. 7F, 7G).

Also in mouse bone marrow derived macrophages (BMDM), a highly relevant model to investigate innate immune signaling *in vitro,* Dox induced the expression of ISGs and triggered the phosphorylation of TBK1 (Fig. 1I-J). mtDNA effusing from the mitochondria into the cytosol was shown before to elicit the type I IFN response in the context of mitonuclear genomic instability caused by the loss of function of Transcription Factor A Mitochondrial (TFAM) (*12*). Also increased levels of mtDNA were detected in the cytosol of Dox-treated BMDMs (Fig. 1K), which underpins the activation of antiviral signaling that results in the secretion of IFNβ from these BMDMs (Fig. 1L). Finally, Dox-induced secretion of IFNβ was abrogated by the nucleoside analogue, 2',3'-dideoxycytidine (ddC) (Fig. 1L), which gradually leads to depletion of mtDNA (*24*), demonstrating that cytosolic release of mtDNA contributes to the activation of antiviral signaling.

It was then set out to identify tetracyclines with lower antimicrobial activities that could be easier developed for clinical use. To this effect, in *C. elegans* a library of 51 position modified Tet derivatives was then screened that are clinically used, are synthetic intermediates, or derivatives specifically synthesized to probe initial structure-activity relationships (SAR) amongst Tets that elicit the MSR (Fig. 2A-C), most of them having very limited antibacterial activity (Fig. 2B).

Compound 1 of 6 of the 51 compounds are shown in Fig. 2 and compounds 6 to 51 in the following Table 1. Compounds not encompassed by the general formulae (I) and (II) as defined in the claims are reference compounds.

**Table 1**

| | **No. Source** | **Name** | **Activity in Worm Assay** | **Synthesis Method or** |
|---|---|---|---|---|
| 6 | 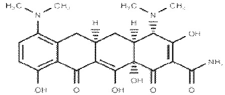 | minocycline | **inactive** | Provided by Hovione, Sete Casas 2674 - 506 Loures, Portugal |
| 7 | 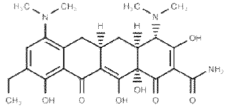 | 9-ethyl minocycline | inactive | **B** |
| 8 | 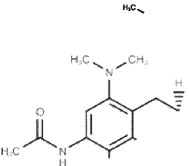 | 9-acetyl aminominocycline | inactive | **D** |
| 9 | 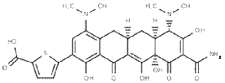 | 9-(4'-carboxy-1'-thiophene) minocycline | inactive | **B** |
| 10 | 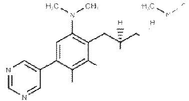 | 9-(5'-pyrimidyl) minocycline | inactive | **B** |
| 11 | 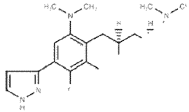 | 9-(3'-pyrazolyl) minocycline | inactive | **B** |
| 12 | 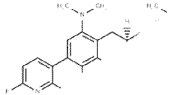 | 9-(3'-pyridinyl-2,6-difluoro) minocycline | inactive | **B** |
| 13 | 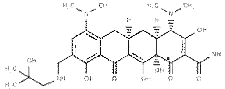 | Omadacycline | inactive | **C** |
| 14 | 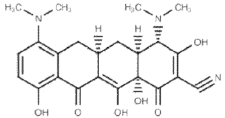 | 2N nitrile minocycline | inactive | **F** |
| 15 | 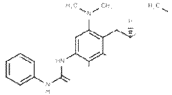 | 9-(phenylurea) aminominocycline | inactive | **J** |
| 16 | 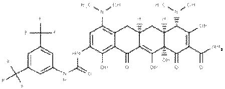 | 9-(3,5-trifluoromethylphenylurea) aminominocycline | inactive | **J** |
| 17 | 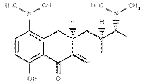 | 2N-1,1-dimethylbutane minocycline | slight | **F** |
| 18 | 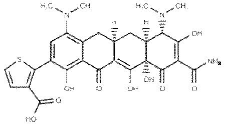 | 9-(2'carboxy-1'-thiophene) minocycline | inactive | **B** |
| 19 | 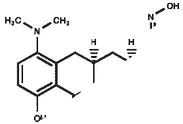 | 4-oxime minocycline | inactive | **J** |
| 20 | 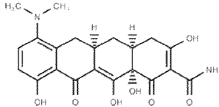 | 4-dedimethylamino minocycline | inactive | **B** |
| 21 | 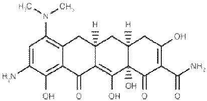 | 4-dedimethylamino-9-aminominocycline | inactive | **B, D** |
| 22 | 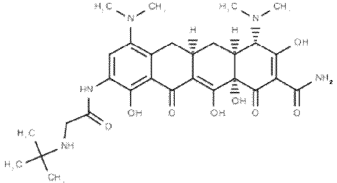 | Tygacil^{™} | slight | **D** |
| 23 | 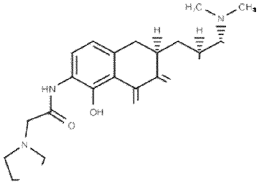 | 9-(pyrrolidinylmethylcarbonyl) aminominocycline | inactive | D |
| 24 | 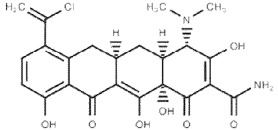 | 7-(1-chlorostyryl) sancycline | inactive | **B** |
| 25 | 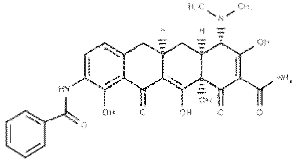 | 9-phenacyl aminosancycline | inactive | **D** |
| 26 | 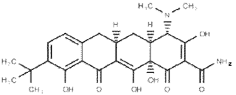 | 9-tert-butyl sancycline | inactive | **G** |
| 27 | 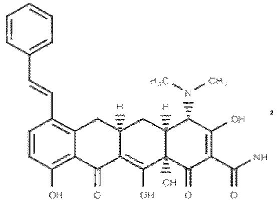 | 7-(1-styrylphenyl) sancycline | inactive | **B** |
| 28 | 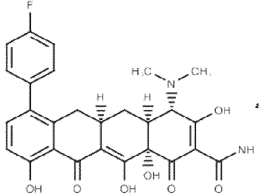 | 7-(4-fluorophenyl) sancycline | inactive 32 | **B** |
| 29 | 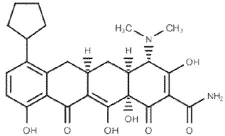 | 7-cyclopentyl sancycline | inactive | **B** |
| 30 | 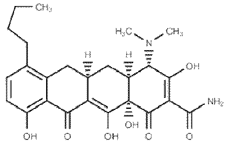 | 7-butyl sancycline | slight | **B** |
| 31 | 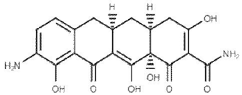 | 4-dedimethylamino-9-amino sancycline | inactive | **B** |
| 32 | 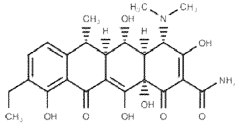 | 9-ethyl doxycycline | inactive | **B** |
| 33 | 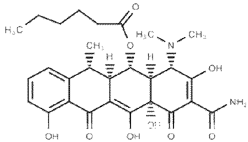 | 5-hexanoate doxycycline | slight | **G** |
| 34 | 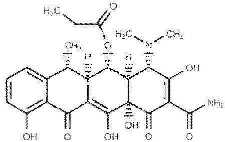 | 5-propionate doxycycline | inactive | **G** |
| 35 | 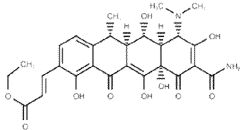 | 9-ethylacrylate doxycycline | slight | **B** |
| 36 | 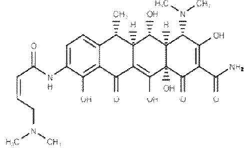 | 9-(3'-dimethylaminoacrylate) amino doxycycline | inactive | **D** |
| 37 | 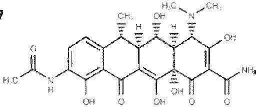 | 9-acetylamino doxycycline | inactive | **D** |
| 38 | 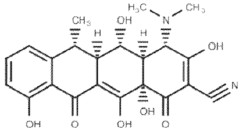 | 2N-nitrile doxycycline | inactive | **F** |
| 39 | 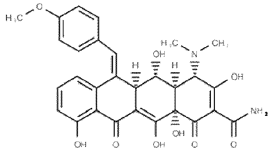 | 13-(4'-methoxyphenyl) methacycline | slight | **B** |
| 40 | 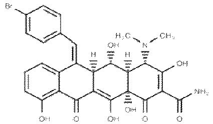 | 13-(4'bromophenyl) methacycline | slight | **B** |
| 41 | 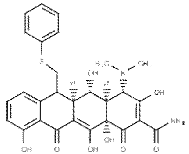 | 13-thiophenyl dihydro methacycline | slight | **A** |
| 42 | 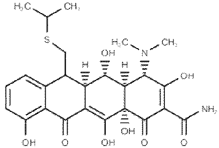 | 13-isopropylthio dihydro methacycline | slight | **A** |
| 43 | 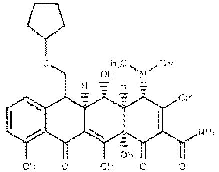 | 13-cyclopentylthio dihydro methacycline | slight | **A** |
| 44 | 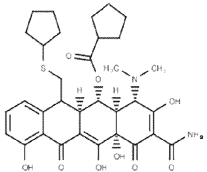 | 5-cyclopentanoate-13-cyclopentylthio dihydro methacycline | slight | **A, G** |
| 45 | 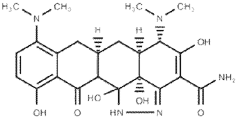 | 1,12- hydroxypyrazolo minocycline | inactive | **H** |
| 46 | 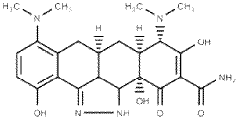 | 11,12-pyrazolo minocycline | inactive | **I** |
| 47 | 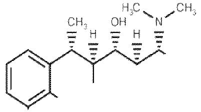 | 11,12-pyrazolo doxycycline | inactive | **I** |
| 48 | 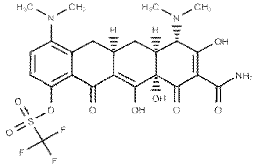 | 10-triflate minocycline | inactive | **Unpublished results** |
| 49 | 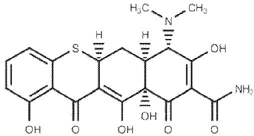 | Thiatetracycline | inactive | **Gift from Pfizer, Inc. Groton, CT USA** |
| 50 | 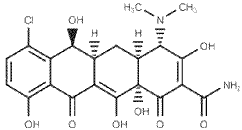 | demeclocycline | slight | **Gift from Pfizer, Inc. Groton, CT USA** |
| 51 | 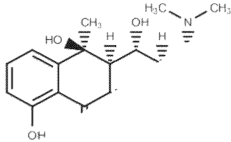 | oxytetracycline | slight | **Gift from Pfizer, Inc. Groton, CT USA T USA** |

Compounds 1-39, 41-59, and 50, 51 were prepared as the HCl salt forms.

The compounds were screened for UPR^{mt} induction using a C. elegans *hsp-6::gfp* reporter strain with Dox administration and cco-1 RNAi feeding as positive controls, as previously described (*25*) (Fig. 2D). Out of the 51 Tet derivatives tested, representing clinically relevant and C2-C10 position modified compounds (26-32) (Fig. 2A-B), 9-tert-butyl-doxycycline **1** (9-TB) and anhydrotetracycline **2** (ATc) were identified as the strongest activators of the UPR^{mt} (Fig. 2C-D). Detailed dose-responses of Dox, 9-TB, and ATc to induce a GFP signal in the *C. elegans hsp-6::gfp* reporter strain were then compared in an automated microfluidic device (33). 9-TB and ATc were again in this system more efficacious at lower doses to induce the UPR^{mt} relative to Dox (Fig. 2E), with 9-TB surpassing the robust UPR^{mt} caused by *cco-1* RNAi feeding (Fig. 2D, 2E).

In addition, other derivatives modified along the upper periphery spanning positions 2, C4, C5, C6, C13 and aromatic positions C7-C9 also activated the UPR^{mt}, such as compounds **3-5,** although their effect was not as pronounced as that of 9-TB and ATc (Fig. 2A-B). In contrast, clinically used minocycline **7,** Nuzyra^{™} **14,** Tygacil^{™} **23** or derivatives based on the minocycline scaffold did not activate the UPR^{mt} (16 compounds), while C5-C-9 derivatives of sancycline only mildly activated the UPR^{mt}. Additionally, compounds modified at the lower periphery, spanning positions C10, C11, C12-C1, and the A-ring C2 carboxamide did not induce the activity of the GFP reporter, showing the importance of this integrated phenolic keto-enol system in maintaining UPR^{mt} activity (*34*, *35*).

The pharmacology of Dox, 9-TB and ATc was then characterized in human embryonic kidney (HEK) 293T cell line. 9-TB and ATc also generated a more robust MSR response than Dox, as reflected by their impact (up to almost 2-fold stronger) on the mitonuclear protein imbalance (Fig. 3A), a disbalanced ratio between mitochondrial- *versus* nuclear-encoded OXPHOS subunits, underpinning the induction of the MSR (*5*). Furthermore, 9-TB and ATc reduced basal oxygen consumption rate (OCR) in a dose-dependent and more pronounced fashion than Dox (Fig. 3B). The induction of transcripts for the mammalian MSR signature genes, was likewise more prominent with 9-TB and ATc (Fig. 3C). In mouse BMDMs, lower doses of 9-TB (1.88 µg/ml) and ATc (3.75 and 7.5 µg/ml) were also superior to Dox (at 7.5-15 µg/ml) to induce the ISGs and MSR genes (Fig. 3D, 8A) and the secretion of IFNβ (Fig. 3E). Taken together, these studies in *C. elegans,* mouse BMDMs, and human HEK293T cells ascertained the identification of non-antimicrobial Tets with higher potency to trigger the MSR and type I IFN response, relative to our benchmark anti-bacterial Tet, Dox.

mtDNA instability-driven innate immunity can potentiate resistance to viruses (*12*) and mediates the antiviral immune response against the IFV (36). It was asked whether the Tet-induced MSR enables mice to survive infection by IFV. Hence, 8 week-old female BALB/cN mice were subjected to either mock (1 group, n=10) or intranasal inoculation with 175 PFU of the IFV H1N1 PR8 strain (3 groups). The 3 infected groups (n=10 each) were given vehicle, Dox (at 40 mpkd) or 9-TB (at 1 mpkd) by daily intraperitoneal injection (Fig. 9A). Dox and 9-TB improved the survival to the infectious challenge, with 50% of the mice treated with Dox or 9-TB recovering (Fig. 4A). The improved health of the Tet-treated cohorts was further supported by the recovery of body weight loss, and their improved clinical score (Fig. 4B, 9C). In contrast, the IFV infection was lethal to all control mice by day 11 post inoculation. Strikingly, at day 7 post infection no significant difference in viral titer in the lung tissue was observed (Fig. 4C). Similarly, when mice were infected with a much higher viral load (1000 PFU; Fig. 9B), Dox and 9-TB delayed mortality and the decline in health (Fig. 4E-F, 9E), again in the absence of an impact on the viral titer in the lungs at day 5 post-infection (Fig. 4G). The Tet-induced MSR did not cause obvious adverse effects (Fig. 9D), yet decreased the levels of Interleukin 6 (IL-6) in both 175 and 1000 PFU experiments (Fig. 4D, 4H), and of some other markers of tissue stress and damage (Fig. 9F) (37-39). These results demonstrate that the Tet-induced MSR increases the survival of mice to a lethal IFV by improving tolerance, rather than by reducing viral load, which is reflective of resistance to the virus.

To gain insight into the mechanisms underlying the Tet-induced disease tolerance the transcriptomes of the lung was analyzed, as well as of liver and kidney (Fig. 10A), 2 organs often affected by the multiorgan failure syndrome seen after infection by respiratory viruses like IFV or SARS-CoV2 (40). In each tissue, principal component analysis (PCA) separated non-infected from IFV infected mice along the first dimension, PC1, whereas 9-TB had a more pronounced and less variable effect, with better clustering and further separation, along the second dimension relative to control and Dox transcriptomes (Fig. 5A, 10B).

GSEA showed that 9-TB significantly down-regulated multiple inflammatory and immune-related terms in the lungs (Fig. 5B, 10D), such "immune response", "t cell activation" or "b cell activation". It was then sought to characterize how 9-TB reversed the effect of IFV infection on transcript levels (Fig. 10A). It was thus assessed which Gene Ontology (GO) Biological Processes (GOPB) terms were enriched in the intersection of gene sets changed in opposite directions by infection and 9-TB, respectively (Fig. 10C). As summarized by Revigo analysis (*41*) (Fig. 10A), inflammatory, immune and apoptotic processes were the main enriched terms amongst genes induced by IFV and down-regulated by 9-TB (Fig. 5C, left panel). Infection by IFV leads to lung epithelial cell dysfunction and down-regulation of genes implicated in cilia or tight junctions, which underpin failures of muco-ciliary clearance and barrier function that contribute to the pathogenesis of ARDS (*19*). Accordingly, multiple gene sets related to lung development and to lung cell function and structure were decreased by IFV infection and their expression was restored by 9-TB (Fig. 5B, 10E, 5C, right panel). Altogether, 9-TB elicits disease tolerance to IFV mainly by counteracting inflammation and the loss of lung epithelial cells and structures, processes that directly determine the severity of infection by respiratory viruses.

To estimate the impact of the infectious challenge or Tet treatment on the lung cell types, extant single-cell RNA-Seq (scRNA-Seq) transcriptomic profiles of mouse (*42*) and human (*43*) lung cell populations from 2 independent studies studying IFV infection were reanalyzed; one of these studies furthermore established cell markers that overlap between mouse and human lung cell types (*43*). Using these cell profiles to perform GSEA on our data confirmed that 9-TB reverted the loss of multiple cell types crucial to lung function, such as club, ciliated and alveolar epithelial cells (Fig. 5D), whereas it decreased several classes of immune cells, such as neutrophils, natural killer cells and monocytes, all contributing to tissue damage upon IFV infection (*17*). Dox showed similar, but more discrete, tendencies towards changes in cellular patterns (Fig. 5D). All these observations were confirmed when using a different set of scRNA-Seq cell profiles on IFV infected mouse lungs (Fig. 11A) (42).

9-TB also down-regulated multiple immune-related and inflammatory gene sets in liver and kidney (Fig. 11B). In particular, as shown through Revigo analysis, these immune and inflammatory terms were enriched amongst the group of liver genes induced by IFV and down-regulated by 9-TB (Fig. 11C-D). In the 3 organs studied, Dox was leading to a weaker down-regulation of many of these terms as shown by GSEA (Fig. 11B), suggesting that it does not lower systemic IFV-driven inflammation as efficiently as 9-TB, which is also consistent with its more moderate impact on IL-6 plasma levels (Fig. 4D). On top, Dox induced gene sets involved in cytopathic processes and fibrosis in liver and kidney (Fig. 11B), suggesting an improved safety profile of the 9-TB, relative to Dox, at doses showing similar efficacy. Taken together, our transcriptomic data highlight that Tet-induced mitohormesis (and in particular 9-TB), elicits disease tolerance to IFV by preventing IFV-associated lung damage and by dampening inflammatory responses not only in lungs, but in liver and kidney as well.

One particular point of interest is the inverse relationship between mitochondrial function and type I IFN signaling that was highlighted in Dox-treated livers, kidneys and BMDMs (Fig. 1A-L, 7F-G). A similar anti-correlation between mitochondria-encoded genes, a proxy for both mitochondrial content and functionality, and type I IFN genes was reported in multiple cell types (*44*, *45*), including in IFV-infected mouse lungs analyzed by single-cell profiling (*42*). This data is coherent with our observations that suggest that a mild MSR, as induced by Tets, is adaptive and linked with a moderate and beneficial type I IFN response. High levels of mitochondrial stress that lead to mitophagy, accompanied by a stronger decrease in mitochondrial proteins and mitochondria-encoded transcripts (*21*, *44*), however, might cause overactivation of the IFN response. Impairments in type I IFN signaling constitutes a major risk factor to develop severe forms of COVID-19, low type I IFN activity correlating with high inflammatory cytokines levels (*46*). Human single-cell RNA-Seq data from peripheral blood mononuclear cells from COVID-19 patients (38) also highlighted a highly significant negative correlation between the mean expression of "type I IFN response" GO term and that of mitochondrial genes across all cell types (Fig. 6A). Negative correlations in each cell type separately (Fig. 12A) were most prominent in natural killer, plasmacytoid dendritic cells, CD14+ and CD16+ monocytes, and were more pronounced in severe compared to moderate COVID-19 cases (Fig. 6B). These data hence bring to light an evolutionary conserved anti-correlation between type I IFN signaling and mitochondrial genes as well as the importance of mitochondrial homeostasis upon infection by respiratory viruses, such as IFV, SARS-CoV2.

### Example 2 - discussion

Herein it is reported that Tets can be used to safely induce an adaptive mitohormetic response, leading not only to the activation of the ATF4-ISR pathway, but also to the induction of type I IFN signaling *in vitro* and in germ-free mice. This translates into a beneficial impact on lethal IFV infection, where Tets enabled survival and induced disease tolerance of infected mice. RNAseq data from lung, liver, and kidney helped to unveil the mechanisms underlying tolerance to IFV infection. Tets rescued the transcript levels of genes involved in lung epithelial cell function and implicated in cilia or tight junctions, which are often found downregulated upon viral respiratory infections as a result of lung damage (19). On the contrary, Tets systematically down-regulated multiple inflammatory and immune related gene sets in the lung, liver, and kidney transcriptome data (Fig. 5, 11). Enrichment of cell profiles based on scRNA-Seq suggested that Tets attenuated the loss of club, ciliated and alveolar epithelial cells in the lungs, while reducing immune cell infiltration (Fig. 5D). Also a robust reduction of IL-6 (Fig. 3D, H) and of other markers of inflammation and tissue damage (Fig. 9D). Although the detailed kinetics of the immune response to IFV remain to be demonstrated, it is speculated that a possible mechanism for Tet-induced mitohormesis may involve a mild boost of the IFN response early after treatment, leading to overall favorable consequences on inflammation and tissue damage (*47*), as explained below.

One important limitation for the use of Tets, which inhibit both the bacterial and hence also the mitochondrial translation, comes from their anti-microbial activity that influences the host microbiome. Using a primary screening that identified compounds based on the induction of the UPR^{mt} in C. *elegans,* combined with the analysis of their anti-microbial activity, a series of candidates was identified through preliminary SAR that have no or very weak antimicrobial activity, yet retain full or even superior capacity to induce the MSR. Indeed, lower doses of 9-TB and ATc led to the induction of the MSR *in vitro* and to disease tolerance *in vivo;* these compounds were devoid of some of the adverse effects of Dox (Fig. 11). This indicates that one can select compounds for their enhanced activity on the mitobiome *versus* the microbiome and thereby dial out some adverse effects of the Tets. Future work will have to further decouple their effect on mitohormesis from other pleiotropic effects through which Tets impact on physiology (*48*). Yet, a recent report in line with our results demonstrated that genetically induced mitohormesis also triggers disease tolerance to bacterial sepsis, and underscores the prime importance of the MSR in inducing disease tolerance (*14*).

Mild levels of mitochondrial damage or dysfunction have the potential to activate type I IFN or other immune pathways, leading to inflammation (*49*). It hence appears logical that immune and mitochondrial quality control pathways are co-regulated in interlocked feedback circuits to prevent mitochondrial damage upon inflammatory triggers and *vice versa.* The anti-correlation between IFN- and Mito-modules highlighted in single-cell data for both IFV (*42*) and COVID-19 (Fig. 6) (*38*) supports this hypothesis. This also indicates that moderate mitochondrial stress could be adaptive and trigger the induction of low levels of type I IFN that are beneficial (*50*). A finely tuned IFN response allows a balanced immune response with optimal protection and minimal tissue damage, limiting the detrimental effect of IFN (*51*). As an example, in COVID-19, endogenous high levels of type I IFN as well as early administration of IFNα decreased mortality while late administration of IFNα increased mortality (52); this is consistent with the fact that delayed and uncontrolled IFN responses disrupt lung repair and induce immunopathology (*53*, *54*). Given the importance of a balanced type I IFN response in the course of IFV and COVID-19 infection, the cross-species anti-correlation with mitochondrial genes highlighted here advocates for mitochondrial homeostasis as a central target for the management of respiratory viral infections.

Future investigations will have to determine how mitochondrial quality control and innate immune pathways mechanistically combine to translate into Tet-induced disease tolerance to IFV and potentially other viral infections. The ensuing insight may not only open new therapeutic avenues to cope with infections by respiratory viruses, but also to manage other diseases typified by mitochondrial dysfunction and inflammation, such as neurodegenerative (e.g. Alzheimer's (55)) and cardiovascular diseases (e.g. aortic aneurism (56)).

### Example 3 - Materials and Methods

### List of abbreviations

9-TB: 9-tert-butyl-doxycycline
ARDS: acute respiratory distress syndrome
ATc: anhydrotetracycline
BMDM: bone marrow-derived macrophages
COVID-19: coronavirus disease 2019
Dox: doxycycline
GSEA: gene set enrichment analysis
GO: gene ontology
IFN: interferon
IFV: Influenza virus
ISR : integrated stress response
OXPHOS: oxidative phosphorylation
MODS: multiorgan dysfunction syndrome
MSR: mitochondrial stress response
mtDNA: mitochondrial DNA
PBMC: peripheral blood mononuclear cell
SARS-CoV2: severe acute respiratory syndrome coronavirus 2

### Study design

The initial goals of the study were to characterize the transcriptional response to Dox-induced mitochondrial stress in germ-free C57BL/6 mice (overcoming the confounding factors of flora disturbances) and to screen for potent, non-microbial Tets inducing the UPR^{mt} in *C. elegans.* HEK293T and BMDM cells were used to validate the initial findings *in vitro.* The most promising compounds, 9-TB, was tested in comparison with Dox for its efficacy in conferring disease tolerance to mice infected by IFV A in 2 different studies with different initial viral inoculation loads. Lungs, liver and kidneys of 5-6 IFV infected mice were recovered at day 7 post-inoculation, RNA was extracted and analysed by RNA-Seq. Finally, single-cell data of PBMCs from COVID-19 patients (*38*) were reanalysed to assess the correlation between type I IFN genes (GO:0034340) and mitochondria-encoded genes. All animals used in the experiments were randomly assigned to experimental or control groups. Animals' care was in accordance with institutional guidelines. All animals that showed signs of severe illness, predefined by the animal authorization protocol before the start of the experiment, were euthanized. Mouse experiments were performed once. IFV infection studies and the *C*. *elegans* screening were performed in a blinded manner. Calculation of sample sizes for worm, cell and animal experiments were determined based on previous findings. Sample sizes, replicates, and statistical methods are specified in the figure legends.

### Statistical analyses

Differences between two groups were assessed using two-tailed t tests. Differences between more than two groups were assessed with one-way analysis of variance (ANOVA), unless stated otherwise. For survival curves, statistical analysis was performed by Log-rank (Mantel-Cox) test. GraphPad Prism 6 was used for statistical analyses. Variability in plots and graphs is presented as standard error mean (SEM), unless stated otherwise. All p ≤ 0.05 were considered to be significant. *p ≤ 0.05; **p ≤ 0.01; ***p ≤ 0.001.

### Mouse experiments in C57BL/6J mice

Male 9 weeks-old C57BL/6J mice were treated for 16 days with 500 mg/kg/day doxycycline hyclate (Sigma) in drinking water. Mice were housed with ad libitum access to water and food and kept under a 12-hour dark/12-hour light cycle. As doxycycline is bitter the water was supplemented for both conditions (treatments and controls) with 50 g/L sucrose. Drinking water was changed every 48 hours. Germ-free C57BL/6J mice were obtained from the Clean Mouse Facility, University of Bern (Bern, Switzerland), and compared with specific pathogen-free (SPF) C57BL/6J mice from Janvier Labs. All animal experiments were carried out according to the institutional, national Swiss and EU ethical guidelines and were approved by the local animal experimentation committee of the Canton de Vaud.

### IFV infection in BALB/cN

8 weeks-old female mice were inoculated on day 0 with influenza virus A (Influenza A/PR/8/34 (H1N1) originating from ATCC VR-1469) via intranasal route at the amount of 175 PFU/mouse/50µL, or 1000 PFU/mouse/50µL respectively (for the high viral challenge study) (Fig. 9A, 9B), under anesthesia by IP injection of anesthetic (Zoletil 50 at 30 mg/kg+Xylazine at 6 mg/kg). Mice in all groups were treated with vehicle (saline) or the indicated concentrations of Dox/9-TB by intraperitoneal injection from day -3 (respectively day -7) until death/sacrifice (Fig. 9A, 9B). Body weight was monitored during the whole study. Body temperature, food intake (daily consumption in each cage) and clinical score were monitored from day 0 until death/sacrifice. For each treatment/control group, 10 mice were followed for survival and 5-6 mice were sacrificed at day 7 (respectively day 5) for blood and organ collection. Blood samples were collected in tubes via cardiac puncture and anticoagulated by K₂EDTA, then centrifuged at 7000 g, 4 °C for 10 minutes to obtain plasma samples. Any mouse suffering from ≥35% body weight loss relative to day 0 was euthanized and counted as dead. Mice were blindly scored on a daily basis as follows: 1 = healthy mouse; 2 = mouse showing signs of malaise, including slight piloerection, slightly changed gait and increased ambulation; 3 = mouse showing signs of strong piloerection, constricted abdomen, changed gait and periods of inactivity; 4 = mouse with enhanced characteristics of the previous grade, but showing little activity and becoming moribund; 5 = mouse found dead. The study was performed by WuXi AppTec (Shanghai) Co., Ltd. according to the protocol following the institutional guidelines of Institutional Committee Animal Care and Use Committee, Shanghai Site (IACUC-SH) and approved by the Shanghai Science and Technology Committee (STCSM, Ministry of Science and Technology, PR of China)

### HEK293T culture and oxygen consumption rate

HEK293T (Human embryonic kidney 293T) cells were purchased from ATCC and have been routinely checked in the laboratory for mycoplasma contamination with the MycoProbe detection kit (R&D systems). HEK293T were grown at 37 °C in a humidified atmosphere of 5% CO₂/95% air in DMEM medium with 4.5g/L glucose (Gibco) including 10% FBS (Gibco), 1× nonessential amino acids (Invitrogen) and 5 mM penicillin/streptomycin (Invitrogen). Cells were treated for 24h with the indicated doses of compounds 24 hours after seeding. Oxygen consumption rate was measured with the Seahorse XF96 instrument (Seahorse Bioscience) according to the manufacturer's protocol.

### Isolation and culture of primary murine BMDM

BMDMs were isolated from the femurs and tibias of 10-week-old C57BL/6 mice. Cells were plated on bacteriological plastic plates in macrophage growth medium consisting of RPMI-1640 (Invitrogen), 1×HEPES (Invitrogen), 5 mM penicillin/streptomycin (Invitrogen), and 10% heat-inactivated fetal bovine serum (Gibco) supplemented with 15% L-cell-conditioned medium as a source of CSF-1. After 1 day, nonadherent cells were collected, seeded at 8×10⁵ cells/ml in bacteriological plates, and grown for 5 more days.

### Western blot

Tissues and cells were lysed using RIPA buffer (50 mM Tris-HCl pH 7.4, 150 mM NaCl, 1% NP-40, 0.5% Na-deoxycholate, 0.1% SDS, 2 mM EDTA, and 50 mM NaF) supplemented with protease and phosphatase inhibitor cocktails (Roche / Thermo Fisher Scientific). Lysates were incubated on ice and cleared by centrifugation at 18,500 g for 15 minutes at 4°C. Protein concentration was determined by the Lowry method. Proteins were separated by SDS-PAGE and transferred onto polyvinylidene difluoride membranes. Proteins were detected using commercial antibodies against eIF2α, phospho-eIF2α (both from Cell Signaling), HSP90 (Santa Cruz), ASNS (Atlas antibodies), HSPA9 (Antibodies Online), LONP1 (Sigma), OXPHOS proteins (Total OXPHOS Rodent WB Antibody Cocktail, Abcam) and β-tubulin (Santa Cruz). Samples were analyzed by immunoblotting using standard procedures.

### Microarray analysis and GSEA

Total RNA was isolated from flashfrozen and powdered liver and kidney aliquots using TRIzol (Life Technologies). RNA was purified using the RNeasy Mini Kit (Qiagen) in accordance with the manufacturer's instructions. Microarray analysis was performed using Affymetrix *mouse MAT1.0* chips in triplicates for each condition. Microarray data was normalized with RMA-sketch method of the Affymetrix Expression console and analyzed using limma R package (*57*). Bonferroni adjusted p value<0.05 was used to determine the differentially expressed genes. GSEA was performed using the clusterProfiler package (*58*). Genesets in gmt format were obtained from the MSigDB Collections from the Broad Institute website. For each organ, all expressed genes were ordered by decreasing fold change based on the differential expression analysis upon Dox. 10'000 permutations, a minimum gene set size of 10, and a maximum of 1000 were performed.

### RNA-Seq analysis and GSEA analysis

RNA sequencing analysis was performed with extracted RNA from mouse tissues (Lungs, Liver & Kidneys) recovered at day 7 post intranasal infection with 175 PFU with influenza virus A (Influenza A/PR/8/34 (H1N1) originating from ATCC VR-1469) (n=5-6). RNA was extracted from flashfrozen, powedered tissue aliquots and cleaned using TRIzol reagent (Invitrogen) followed by Direct-zol-96 RNA kit (Zymo Research). RNA quality was assessed using Fragment Analyzer (Agilent). 1 ug of total RNA was used for the construction of sequencing libraries. For each sample, 60 million paired-end sequencing reads of length of 100 base pairs each were sequenced using DNBseq Eukaryotic-T resequencing (BGI Sequencing). FastQC (*59*) was used to verify the quality of the mapping. No low-quality reads were present and no trimming was needed. Alignment was performed against mouse genome (CRCm38 mm10 primary assembly and Ensembl release 95 annotation) using STAR (version 2.73a) (*60*). The obtained STAR gene-counts for each alignment were analyzed for differentially expressed genes using the R package edgeR (version 3.24.3) (*61*) using a generalized-linear model. A threshold of 1 log2 fold change and adjusted P value smaller than 0.05 were considered when identifying the differentially expressed genes. A principal component analysis was used to explore the variability between the different samples. The RUVSeq (version 1.16.1) (*62*) Bioconductor R package was used to correct for the unwanted variation using a set of empirical control genes (all but top 5000 most significantly expressed genes determined from a first-pass differential expression analysis). The clusterProfiler R package was used to conduct GSEA analysis on gene ontology (GO) terms (*58*). A minimum gene set size of 10, a maximum gene set size of 500 were used, and 10,000 permutations were performed. A gene list ordered by log2(Fold Changes) was used from the differential expression analysis. The clusterProfiler (version 3.17.1) and enrichplot (version 1.9.1) packages were used for GSEA and various data representations.

### qRT-PCR

RNA from cells and tissues was extracted using TRIzol and then reverse-transcribed into cDNA by the QuantiTect Reverse Transcription Kit (Qiagen) following the manufacturer's instructions. The qPCR reactions were performed using the LightCycler 480 II system and SYBR Green qPCR Master mix (Roche). All results are presented relative to the mean of *housekeeping genes* (ΔΔ*C*ₜ method). All mRNA expression levels were corrected for expression of the housekeeping gene *36B4* or *Actb* for samples of mouse origin, *ACTB* for samples of human origin.

The following primers were used for RT-qPCR:

| **gene** | **forward** | **reverse** |
|---|---|---|
| **Mouse** | | |
| ***Asns*** | TTGACCCGCTGTTTGGAATG | CGCCTTGTGGTTGTAGATTTCAC |
| ***Chop*/*Ddit3*** | CGGAACCTGAGGAGAGAGTG | CGTTTCCTGGGGATGAGATA |
| ***Lonp1*** | ATGACCGTCCCGGATGTGT | CCTCCACGATCTTGATAAAGCG |
| ***Hspa9*** | AATGAGAGCGCTCCTTGCTG | CTGTTCCCCAGTGCCAGAAC |
| ***Hspe1*** | CTGACAGGTTCAATCTCTCCAC | AGGTGGCATTATGCTTCCAG |
| ***Cxcl10*** | CCACGTGTTGAGATCATTGCC | GAGGCTCTCTGCTGTCCATC |
| ***Ifi44*** | CTGATTACAAAAGAAGACATGACAGAC | AGGCAAAACCAAAGACTCCA |
| ***Ddx58*** | ATGTGCCCCTACTGGTTGTG | CCCCAGAAATGCTCGCAATG |
| ***Ifit3*** | TTC CCA GCA GCA CAG AAA C | AAA TTC CAG GTG AAA TGG CA |
| ***Irf7*** | CAA TTC AGG GGA TCC AGT TG | AGC ATT GCT GAG GCT CAC TT |
| ***36B4*** | AGATTCGGGATATGCTGTTGG | AAAGCCTGGAAGAAGGAGGTC |
| ***Actb*** | GAGACCTTCAACACCCC | GTGGTGGTGAAGCTGTAGCC |

| **Human** | | |
|---|---|---|
| ***HSPA9*** | TGGTGAGCGACTTGTTGGAAT | ATTGGAGGCACGGACAATTTT |
| ***LONP1*** | CCCGCGCTTTATCAAGATT | AGAAAGACGCCGACATAAGG |
| ***ASNS*** | ATCACTGTCGGGATGTACCC | TGATAAAAGGCAGCCAATCC |
| ***CHOP*** | ACAGTGTCCCGAAGGAGAAAGG | GCCAAAATCAGAGCTGGAACCT |
| ***ACTB*** | TCGTGCGTGACATTAAGGAG | GTCAGGCAGCTCGTAGCTCT |

### Complex activity measurements

Starting from powdered tissues, respiratory chain complex activities were measured by Metabiolab (France/Belgium) as described in (*63*). The activities of all the complexes in each sample were normalized by the amount of protein or referred to citrate synthase activity to allow sample comparison.

### - Complex I activity measurement

Briefly, reduced nicotinamide adenine dinucleotide phosphate (NADPH)-ubiquinone reductase activity (complex I) was measured by following the disappearance of NADPH using rotenone as a specific inhibitor to ensure the specificity of the assay.

### - Complex II activity measurement

Complex II activity, succinate-ubiquinone reductase, was assayed through the reduction of 2,6-dichlorophenolindophenol, a final electron acceptor, after the addition of succinate.

### - Complex III activity measurement

The activity of complex III, ubiquinone-cytochrome c reductase, was determined by assaying the rate of reduction of cytochrome c.

### - Complex IV activity measurement

The cytochrome c oxidase (complex IV) activity was based on the same assay as for complex III using potassium cyanide to inhibit its activity.

### - Complex V activity measurement

Complex V activity was measured according to a method coupling ADP production to NADH disappearance through the conversion of phosphoenol-pyruvate into pyruvate then into lactate.

### - Citrate synthase (CS) activity measurement

The activity of CS was assayed as described previously with the reduction of DTNB caused by the deacetylation of acetyl-CoA.

### ATP measurement

Total ATP content was measured by the Cell Titer-Glo luminescent cell viability assays (Promega) in protein lysate of kidney powdered tissue. The luminescence was recorded with a Victor X4 plate reader (PerkinElmer) and values are normalized by the total protein concentration determined using a Bradford assay.

### Quantification of mtDNA released into cytosol

After 1 hour of treatment with the indicated concentration of Dox, day 6-differentiated BMDM (a 10-cm cell culture for n=1) were harvested by gentle incubation in Cell dissociation Buffer (Gibco) (2 min at 37°C), harvested in a tube, briefly centrifuged (400g, 4 min) and rinsed once with PBS. Then, the assessment of cytosolic mtDNA was carried out as described and with the same primers as in (*64*).

### IFNβ measurement in culture medium

BMDM at day 6 of differentiation were treated with the indicated concentrations of drugs in a controlled volume of culture medium for 16-24 hours. Culture medium was harvested and was assessed for IFNβ concentration using the VeriKine-HS Mouse Interferon Beta Serum ELISA kit (PBL assay Science) according to the manufacturer's instructions.

### Compound screening in C. elegans

The strain used to assess UPR^{mt} activation was SJ4100 (zcls13[*hsp*-*6*::GFP]) (*25*) and was provided by the Caenorhabditis Genetics Center (University of Minnesota). Worms were maintained on nematode growth medium (NGM) agar plates seeded with E.coli OP50 at 20 °C. Compound screening plates were obtained by dissolving each compound at 68 µM (except 9-TB at 17µM, for which the effect was too strong at higher concentrations) in NGM agar supplemented with carbenicillin (25mg/L) and IPTG (2mM) and seeded with HT115 RNAi control bacteria or with *cco-1* RNAi clone (F26E4.9). L4 larvae were transferred manually on the compound screening plates and fluorescence was assessed at day 1 of adulthood (similar exposure time for all images). The screening was performed at 20°C.

### C. elegans experiments in microfluidic system

UPR^{mt} activation in the nematode *C*. *elegans* was studied using microfluidic-based testing protocols on an automated platform, as built and optimized by Nagi Bioscience SA (33). Briefly, L1 larvae were harvested in complete S-medium and injected into microfluidic chips. Worms were then continuously fed on-chip via bacterial medium, for the duration of the entire experiment. Each tested chemical was added to a 350 µL aliquot of bacterial medium, at 5 concentrations. 4 µL of fresh food/chemical solution were injected into the microfluidic chip per each tested condition every 60 min. Fluorescent pictures of each micro-chamber were acquired after the feeding, every 60 min, during 110 hours. For the fluorescent image acquisition, worms were exposed to blue light for 30 milliseconds. Then, images were analyzed using software algorithms developed by Nagi Bioscience SA, allowing the extraction of the fluorescence intensity per worm. By plotting the average fluorescence intensity over time, the GFP expression kinetic for each condition was plotted. Finally, to quantify the level of GFP expression for each condition, the Area Under the Curve (A.U.C.) of the mean fluorescence intensity was calculated and then the peak fluorescence intensity was extracted for each condition. The experiments were performed at 20 °C.

### Viral titer

The lung viral titer was determined by plaque assay and the data are shown as Log 10 (plaques/ g tissue). The plaque assay was performed with the MDCK cells as follows: MDCK cells were seeded at the density of 2.5*10⁵ cells/mL. Lung samples were homogenised with tissue lyser II after thawing. After centrifugation, the lung homogenates were serially diluted with infection medium, 10-fold for 6 dilutions and pipetted to a 6-well plate. After incubation, the cell infection medium was replaced with infection medium containing 0.625% low-melting-point agarose. After fixation with 4% paraformaldehyde, cells were stained with 0.5% crystal violet solution. Plaques were counted visually and the virus titer was calculated as follows: virus titer/g lung tissue=Log 10 (plaques/well*dilution factor*1000).

### Biochemical parameters measurements

Plasma parameters were measured on 2× diluted samples (1:1 ratio of plasma to diluent) using Dimension^{®}Xpand Plus (Siemens Healthcare Diagnostics AG, Dudingen, Switzerland). The biochemical tests were performed according to the manufacturer kit for each parameters: AST (Siemens Healthcare, DF41A), ALT (Siemens Healthcare, DF143), Total protein (Siemens Healthcare, DF73), Urea Nitrogen (Siemens Healthcare, DF21).

### Plasma cytokines measurement

Plasma cytokines were measured in plasma using the Luminex Mouse Discovery Assay (R&D Systems) according to the manufacturer's protocol.

### Example 4 - 9-TB does not affect the gut microbiome and improves survival from IFV infection also when given therapeutically.

To assess the impact of Tets on the microbiome feces was collected from transiently single-caged animals and longitudinally before (Day -4 pre-IFV-inoculation), 3 days after (Day 0, just before IFV-inoculation) and 6 days after (Day 3, post- IFV-inoculation) the start of daily administration of 9-TB, respectively Dox. Then DNA was extracted from feces and performed whole metagenome sequencing. While the composition and diversity of the bacterial communities showed no differences between groups before treatment, the gut bacterial community of mice treated with Dox showed a significant difference in composition compared to both untreated mice and mice treated with 9-TB both after 3 and 6 days of tetracycline treatment (respectively Day 0 and Day 3 post-inoculation), as assessed by permutational multivariate analysis of variance (perMANOVA) and visualized by non-metric multidimensional scaling (NMDS) **(****Fig. 13A** **and Table 2).** This was reflected by a lower bacterial species diversity in Dox-treated mice both in terms of Shannon diversity index (SDI) and richness **(****Fig. 13B****).** In contrast, no differences were observed between 9-TB treated mice and untreated mice at any time point, suggesting that that the administered dose of 9-TB do not affect the mice gut microbiota *in vivo* **(****Fig. 13A****, B and Table 2).**

**Table 2**

| |
|---|
| TimePoint,"Comparison","R2","p","p.adj","sign.sym." Day -4,"'veh' vs '9TB 1 mpkd' vs 'Dox 40mpkd‴,"0.2542","0.1978","NA","ns" Day 0,"'veh' vs '9TB 1 mpkd' vs 'Dox 40mpkd‴,"0.5166","0.0081","NA","**" Day 3,"'veh' vs '9TB 1 mpkd' vs 'Dox 40mpkd‴,"0.4632","0.0032","NA","**" Day -4,"'veh' vs '9TB 1 mpkd‴,"0.2471","0.1451","0.2601","ns" Day -4,"'veh' vs 'Dox 40mpkd‴,"0.0814","0.6908","0.6908","ns" Day -4,"'9TB 1 mpkd' vs 'Dox 40mpkd‴,"0.2791","0.1734","0.2601","ns" |
| Day 0,"'veh' vs '9TB 1mpkd‴,"0.1693","0.2821","0.2821","ns" Day 0,"'veh' vs 'Dox 40mpkd‴,"0.6212","0.0293","0.0441","**" Day 0,"'9TB 1 mpkd' vs 'Dox 40mpkd‴,"0.4384","0.0294","0.0441","**" Day 3,"'veh' vs '9TB 1mpkd‴,"0.245","0.1137","0.1137","ns" Day 3,"'veh' vs 'Dox 40mpkd‴,"0.558","0.029","0.0847","*" Day 3,‴9TB 1mpkd' vs 'Dox 40mpkd‴,"0.3119","0.0565","0.0847","*" |

To further investigate the clinical relevance of novel Tet derivatives it was focused on 9-TB and 9-TB was administered in a therapeutic mode starting at day 1 post-inoculation of 760 PFU of the IFV H1N1 PR8 strain **(****Fig. 14A****).** Of note, the different death kinetics and survival proportion with regard to the high viral load (760 PFU) used in this therapeutic experiment **(****Fig. 13C-D** **and** **Fig. 14A-D****)** in comparison with the lower viral load (175 PFU) used in the preventive mode are due to the fact that both experiments were run with different viral batches. Nevertheless, although not significant, the trend of 20% survival upon administration of two very low doses of 9-TB (0.025 and 0.05 mpkd) are highly encouraging **(****Fig. 13C-D****)** and suggest that novel Tets derivatives trigger tolerance to IFV in a clinically relevant setting. Future investigations are thus needed to optimize timing and doses of Tet derivatives and refine their therapeutic potential in viral infections.

### Example 5 - Preparation of exemplary compounds as referred to in Examples 1 to 4

### Example 5.1 - 9-tert-butyl doxycycline

To a solution of doxycycline hyclate (29.2 g, 65.8 mmol) in methanesulfonic acid (329 mL) at rt was added t-butanol (113 mL, 6580 mmol) divided into three (38 mL) portions in 4h intervals. After complete by LCMS (approx. 12h, binary solvent system: A: 0.1% trifluoroacetic acid (TFA), B: acetonitrile 0.1% TFA, C18 column, Gradient 0-100% over 10 minutes), the solution was poured into a solution of ice water (1 L). The solution was made basic through the slow addition of the ice water solution to a saturated solution of sodium bicarbonate with vigorous stirring, and the solution diluted with ethyl acetate (750 mL). After separation of layers, the aqueous solution was extracted with ethyl acetate (750 mL). The combined organic layers were dried over anhydrous sodium sulfate and concentrated under reduced pressure to yield a crude product. The product was suspended in methyl-t-butyl ether (200 mL) and triflic acid was added dropwise (3.51 mL, 40.0 mmol). After judged complete by LCMS (approx. 1h), the solution was slowly poured into a saturated solution of sodium carbonate and diluted with EtOAc (200 mL). After separation of layers, the organic layer was dried over anhydrous Na₂SO₄ and concentrated under reduced pressure to afford a yellow solid of 90.6% purity (AUC) in 93.4% yield. Further purification using water-methanol solvent gradients from 0-100% methanol over 30 minutes using low pressure chromatography on C18 or fluorinated divinyl benzene solid phases by preparative HPLC or by methods known in the art resulted in increases in purity above 98% (C18 reverse phase column, linear gradient 0-100%, A: B, 30 minutes), Rt 16.33 min MS (M+H, formic acid solvent): 501.55. ¹H NMR (Methanol d⁴-300 MHZ) d 7.48 (d, 1H) 6.85 (d, 1H), 4.48 (1H), 3.85 (dd, 2H), 2.98-2.9 (m, 4H), 2.75 (m, 1H), 2.6 (m, 3H), 1.6-1.5 (m, 3H), 1.4-1.45 (m, 9H).

### Reference Example 5.2 - (7,9-Bis(2,6-difluoro-4-pyridynyl)-sancycline)

### (A) Preparation of 7,9-Diiodosancyline

2.0 g of sancycline free base was added to 125 mL of concentrated sulfuric acid with stirring and the solution cooled to 0°C. 3.1 g of N-iodosuccinimide was added slowly over 1 hour and the reaction monitored by LCMS. Once completed, the reaction mixture was added to 500 mL of ice water, and extracted with ethyl acetate, the organic layer dried with sodium sulfate and the solvent removed under reduced pressure. The crude product was purified by using low pressure chromatography on C18 or fluorinated divinyl benzene solid phases by preparative HPLC or by methods known in the art resulted in increases in purity above 95% (C18 reverse phase column, Gradient 0-100%, A: B, 30 minutes) in 43.2% yield. LCMS (positive mode, M+H, 0.1% formic acid Solvent A, acetonitrile Solvent B, linear gradient over 30 mins, 0-100 B, C18 reverse-phase column): Rt= 15.6 minutes, (M+H) 667.45 ¹H NMR (Methanol d-4, 300 MHz) d 8.39 (s, 1H), 3.63 (s, 1H), 3.45 (s, 2H), 2.8 (s,7H), 2.35 (m, 2H), 1.6-1.4 (m, 6H).

### (B) Preparation of (7,9-Bis(2,6-difluoro-4-pyridynyl)-sancycline)

7,9-diiodo sancycline 200 mg (0.254 mmol) and 4.0 mg (0.17 mmol) of palladium acetate were added to 20 mL methanol, the solution purged with nitrogen, and the solution warmed to 50 °C. After 10 minutes stirring 123 mg (0.75 mmol) of sodium bicarbonate and 119 mg (0.75 mmol) of 2,6-difluoropyridine-4-boronic acid added. After the reaction was complete as monitored by LCMS, the solution was filtered through Celite and concentrated under vacuum to yield a crude yellow solid. Further purification using water-methanol solvent gradients from 0-100% methanol over 30 minutes using low pressure chromatography on C18 or fluorinated divinyl benzene solid phases by preparative HPLC or by methods known in the art resulted in increases in purity above 95% (C18 reverse phase column, linear gradient 0-100%, A:B, 30 minutes), Rt 13.75 min MS (M+H, formic acid solvent): 641.6 ¹H NMR (Methanol d⁴-300 MHZ) d 8.3 (s, 1H), 6.8-6.6 (m, 4H), 3.48 (1H), 3.25 (s, 2H), 2.85 (s, 8H), 2.40 (m, 2H), 1.4-1.35 (m, 6H).

### Reference Example 5.3 - 7-bromo sancycline

To a solution of sancycline·TFA (20.7 g, 50.0 mmol) in TFA (200 mL) at 0°C was added N-bromosuccinimide (26.7 g, 150 mmol) in portions or a 30 min. period. After 1 hour, the solution was concentrated to a slurry under reduced pressure. The slurry was poured into a vigorously stirring solution of saturated sodium sulfate. After 2h, the resulting suspension was collected on a fine fritted funnel rinsing with the solid with water. The product as a freebase was further dried under high vacuum in the presence of phosphorous pentoxide to afford 10.2 g as a red-orange solid in 61% yield. Low pressure chromatography on C18 or fluorinated divinyl benzene solid phases by preparative HPLC or by methods known in the art resulted in increases in purity above 95% (C18 reverse phase column, linear gradient 0-100%, A: B, 30 minutes). Rt 9.87 min MS (M+H, formic acid solvent): 494.8, ¹H NMR (Methanol d⁴-300 MHz) δ 7.91-7.88 (d, 1H), 6.78-6.74 (d, 1H), 4.14 (s, 1H), 3.1 (s, 6H), 2.48 (m, 1H), 2.25 (m, 1 H), 1.72 (m, 4H), 1.05 (m, 2H).

### Reference Example 5.4 - 8-bromo sancycline

### (A) Preparation of 9-Nitrosancycline.

To a solution of sancycline HCl (78.3 g, 174 mmol) in trifluoroacetic acid (TFA) (438 mL) at 0°C was added conc. H₂SO₄ (369 mL) slowly, then when the temperature has returned to 0°C powdered KNO₃ (18.5 g, 183 mmol) was added in portions over a 1hour period. At completion of reaction as monitored by LCMS the reaction mixture was slowly poured into a rapidly stirring solution of ice-cold diethyl ether (3 L). After 15 minutes, the resulting suspension was collected onto a fine sintered funnel rinsing with diethyl ether (1.5 L) to afford 97 g in 95% yield. The product was sufficiently pure for the subsequent step.

(B) Preparation of 9-Aminosancycline. A 2 L hydrogenation flask with stir bar was charged with 9-nitrosancycline (57.3 g, 100 mmol), MeOH (950 mL), acetic acid (50 mL) and 5% wet Pd(C) (10 g) was flushed with H₂. After the final cycle, the flask was placed under 40 psi of hydrogen for 6 hours. At completion of reaction, the flask was purged with nitrogen, Celite was added then filtered through a plug of Celite rinsing with MeOH. The solution was concentrated under reduced pressure and converted to the HCl salt by addition of 10% HCl in MeOH and then concentrating under reduced pressure. The crude product is used as is in the subsequent step. Alternatively, the reaction can be done at 1 atm of hydrogen gas with yields that are similar.

(C) Preparation of 8-Bromosancycline. To a solution of 8-bromo-9-aminosancycline (33.5 g, 50.0 mmol) in MeOH (160 mL) and TFA (40 mL) at 0°C was added i-amyl nitrite (7.05 mL, 52.5 mmol) dropwise. At completion of diazotization, after approximately 15 minutes, 50% wt H₃PO₂ (54.7 mL, 500 mmol) and Cu₂O (0.72 g, 5.00 mmol) was added, then the solution was allowed to slowly warm to room temperature. After another 4 hours, the solution was diluted with water (1.5 L) and the product was separated by solid-phase extraction on DVB resin. After loading the water solution onto the DVB resin, the product was eluted with CH₃CN +1% TFA. The solution was concentrated under reduced pressure to afford the crude product as a light brown solid in 56.2 % yield. Alternately, the crude reaction mixture can be concentrated under reduced pressure to remove MeOH and TFA. The crude mixture is cooled by an ice-bath and the pH adjusted to pH 7.0. The water solution is extracted 3X with 10% MeOH/EtOAc.¹³ The organic layers are dried over anhydrous Na₂SO₄ and concentrated under reduced pressure. The crude product is purified by preparatory chromatography eluting with a linear gradient using Solvent A = 0.1% TFA and Solvent B 0.1% CH₃CN or by methods known in the art resulted in increases in purity above 95% (C18 reverse phase column, linear gradient 0-100%, A:B, 30 minutes). Rt 9.95 min MS (M+H, formic acid solvent): 494.7, ¹H NMR (Methanol d⁴-300 MHz) δ 7.1 (s, 1H), 6.5 (s, 1H), 4.52 (s, 1H), 2.8-2.5 (m, 4H), 2.32 (m, 6H), 1.7-1,6 (m, 2H).

### Reference Example 5.5 - Anhydrotetracycline

Twenty grams (20.0 g) of tetracycline hydrochloride were heated at 75 C-80 C. with 300 ml of 35% sulfuric acid for one half hour at the end of which time, an equal volume of Water was added, and the solution cooled in an ice-bath for two hours. The precipitate of crude anhydrotetracycline hydrogen sulfate which formed was collected, washed with water and then suspended in 500 ml. of water. There was then added, one liter of ethyl acetate over a period of ten minutes during which time sufficient NaOH was added to maintain the pH of the water phase at 4.5. The ethyl acetate phase was then separated, and the remaining aqueous phase extracted, while maintaining the pH at 4.5, with an additional liter of ethyl acetate. The ethyl acetate extracts were combined, concentrated to dryness and the resulting crude compound produced as a yellow-orange solid. The crude product, purified by using low pressure chromatography on C18 or fluorinated divinyl benzene solid phases by preparative HPLC or by methods known in the art resulted in increases in purity above 95% (C18 reverse phase column, linear gradient 0-100%, A: B, 30 minutes) in 63.1% yield. Rt 10.3 min MS (M+H, formic acid solvent): 427.4, ¹H NMR (Methanol d⁴-300 MHz) δ 7.4-7.2 (m, 2H), 6.82 (d, 1H), 4.67 (d, 1H), 3.1 (m, 1H), 2.95 (m, 1H), 2.31 (m, 3 H), 2.4-2.2 (m, 6H).

### References

1. Q. Zhao, J. Wang, I. V. Levichkin, S. Stasinopoulos, M. T. Ryan, N. J. Hoogenraad, A mitochondrial specific stress response in mammalian cells. The EMBO journal. 21, 4411-9 (2002).
2. A. Mottis, S. Herzig, J. Auwerx, Mitocellular communication: Shaping health and disease. Science. 366, 827-832 (2019).
3. T. Shpilka, C. M. Haynes, The mitochondrial UPR: mechanisms, physiological functions and implications in ageing. Nature reviews. Molecular cell biology (2017), doi:10.1038/nrm.2017.110.
4. R. Bar-Ziv, T. Bolas, A. Dillin, Systemic effects of mitochondrial stress. EMBO reports. 21, e50094 (2020).
5. R. H. Houtkooper, L. Mouchiroud, D. Ryu, N. Moullan, E. Katsyuba, G. Knott, R. W. Williams, J. Auwerx, Mitonuclear protein imbalance as a conserved longevity mechanism. Nature. 497, 451-7 (2013).
6. J. Durieux, S. Wolff, A. Dillin, The cell-non-autonomous nature of electron transport chain-mediated longevity. Cell. 144, 79-91 (2011).
7. V. Sorrentino, M. Romani, L. Mouchiroud, J. S. Beck, H. Zhang, D. D'Amico, N. Moullan, F. Potenza, A. W. Schmid, S. Rietsch, S. E. Counts, J. Auwerx, Enhancing mitochondrial proteostasis reduces amyloid-beta proteotoxicity. Nature (2017), doi:10.1038/nature25143.
8. Y. T. Wang, Y. Lim, M. N. McCall, K.-T. Huang, C. M. Haynes, K. Nehrke, P. S. Brookes, Cardioprotection by the mitochondrial unfolded protein response requires ATF5. American Journal of Physiology-Heart and Circulatory Physiology. 317, H472-H478 (2019).
9. N. Moullan, L. Mouchiroud, X. Wang, D. Ryu, E. G. Williams, A. Mottis, V. Jovaisaite, M. V. Frochaux, P. M. Quiros, B. Deplancke, R. H. Houtkooper, J. Auwerx, Tetracyclines Disturb Mitochondrial Function across Eukaryotic Models: A Call for Caution in Biomedical Research. Cell reports (2015), doi:10.1016/j.celrep.2015.02.034.
10. E. L. Mills, B. Kelly, L. A. J. O'Neill, Mitochondria are the powerhouses of immunity. Nature immunology. 18, 488-498 (2017).
11. M. W. Pellegrino, A. M. Nargund, N. V. Kirienko, R. Gillis, C. J. Fiorese, C. M. Haynes, Mitochondrial UPR-regulated innate immunity provides resistance to pathogen infection. Nature. 516, 414-7 (2014).
12. A. P. West, W. Khoury-Hanold, M. Staron, M. C. Tal, C. M. Pineda, S. M. Lang, M. Bestwick, B. A. Duguay, N. Raimundo, D. A. MacDuff, S. M. Kaech, J. R. Smiley, R. E. Means, A. Iwasaki, G. S. Shadel, Mitochondrial DNA stress primes the antiviral innate immune response. Nature. 520, 553-7 (2015).
13. V. Tiku, M.-W. Tan, I. Dikic, Mitochondrial Functions in Infection and Immunity. Trends in Cell Biology. 30, 263-275 (2020).
14. H. G. Colago, A. Barros, A. Neves-Costa, E. Seixas, D. Pedroso, T. Velho, K. L. Willmann, P. Faisca, G. Grabmann, H.-S. Yi, M. Shong, V. Benes, S. Weis, T. Köcher, L. F. Moita, Tetracycline Antibiotics Induce Host-Dependent Disease Tolerance to Infection. Immunity. 54, 53-67.e7 (2021).
15. L. Almeida, A. Dhillon-LaBrooy, C. N. Castro, N. Adossa, G. M. Carriche, M. Guderian, S. Lippens, S. Dennerlein, C. Hesse, B. N. Lambrecht, L. Berod, L. Schauser, B. R. Blazar, M. Kalesse, R. Müller, L. F. Moita, T. Sparwasser, Ribosome-Targeting Antibiotics Impair T Cell Effector Function and Ameliorate Autoimmunity by Blocking Mitochondrial Protein Synthesis. Immunity. 54, 68-83.e6 (2021).
16. A. Iwasaki, P. S. Pillai, Innate immunity to influenza virus infection. Nature Reviews Immunology. 14, 315-328 (2014).
17. T. Flerlage, D. F. Boyd, V. Meliopoulos, P. G. Thomas, S. Schultz-Cherry, Influenza virus and SARS-CoV-2: pathogenesis and host responses in the respiratory tract. Nature Reviews Microbiology, 1-17 (2021).
18. M. Singer, The role of mitochondrial dysfunction in sepsis-induced multi-organ failure. Virulence. 5, 66-72 (2014).
19. M. A. Matthay, R. L. Zemans, G. A. Zimmerman, Y. M. Arabi, J. R. Beitler, A. Mercat, M. Herridge, A. G. Randolph, C. S. Calfee, Acute respiratory distress syndrome. Nat Rev Dis Primers. 5, 18 (2019).
20. A. Subramanian, P. Tamayo, V. K. Mootha, S. Mukherjee, B. L. Ebert, M. A. Gillette, A. Paulovich, S. L. Pomeroy, T. R. Golub, E. S. Lander, J. P. Mesirov, Gene set enrichment analysis: A knowledge-based approach for interpreting genome-wide expression profiles. PNAS. 102, 15545-15550 (2005).
21. P. M. Quiros, M. A. Prado, N. Zamboni, D. D'Amico, R. W. Williams, D. Finley, S. P. Gygi, J. Auwerx, Multi-omics analysis identifies ATF4 as a key regulator of the mitochondrial stress response in mammals. The Journal of cell biology. 216, 2027-2045 (2017).
22. D. D'Amico, V. Sorrentino, J. Auwerx, Cytosolic Proteostasis Networks of the Mitochondrial Stress Response. Trends in biochemical sciences. 42, 712-725 (2017).
23. S. Emming, K. Schroder, Tiered DNA sensors for escalating responses. Science. 365, 1375-1376 (2019).
24. I. Nelson, M. G. Hanna, N. W. Wood, A. E. Harding, Depletion of mitochondrial DNA by ddC in untransformed human cell lines. Somat Cell Mol Genet. 23, 287-290 (1997).
25. T. Yoneda, C. Benedetti, F. Urano, S. G. Clark, H. P. Harding, D. Ron, Compartment-specific perturbation of protein handling activates genes encoding mitochondrial chaperones. Journal of cell science. 117, 4055-66 (2004).
26. M. L. Nelson, B. H. Park, J. S. Andrews, V. A. Georgian, R. C. Thomas, S. B. Levy, Inhibition of the tetracycline efflux antiport protein by 13-thio-substituted 5-hydroxy-6-deoxytetracyclines. J Med Chem. 36, 370-377 (1993).
27. M. L. Nelson, M. Y. Ismail, L. McIntyre, B. Bhatia, P. Viski, P. Hawkins, G. Rennie, D. Andorsky, D. Messersmith, K. Stapleton, J. Dumornay, P. Sheahan, A. K. Verma, T. Warchol, S. B. Levy, Versatile and Facile Synthesis of Diverse Semisynthetic Tetracycline Derivatives via Pd-Catalyzed Reactions. J. Org. Chem. 68, 5838-5851 (2003).
28. L. Honeyman, M. Ismail, M. L. Nelson, B. Bhatia, T. E. Bowser, J. Chen, R. Mechiche, K. Ohemeng, A. K. Verma, E. P. Cannon, A. Macone, S. K. Tanaka, S. Levy, Structure-Activity Relationship of the Aminomethylcyclines and the Discovery of Omadacycline. Antimicrob Agents Chemother. 59, 7044-7053 (2015).
29. P. E. Sum, V. J. Lee, R. T. Testa, J. J. Hlavka, G. A. Ellestad, J. D. Bloom, Y. Gluzman, F. P. Tally, Glycylcyclines. 1. A new generation of potent antibacterial agents through modification of 9-aminotetracyclines. J Med Chem. 37, 184-188 (1994).
30. C. R. Stephens, L. H. Conover, R. Pasternack, F. A. Hochstein, W. T. Moreland, P. P. Regna, F. J. Pilgrim, K. J. Brunings, R. B. Woodward, The Structure of Aureomycin1. J. Am. Chem. Soc. 76, 3568-3575 (1954).
31. C. R. Stephens, J. J. Beereboom, H. H. Rennhard, P. N. Gordon, Kotaro. Murai, R. K. Blackwood, M. Schach. von Wittenau, 6-Deoxytetracyclines. IV.1,2 Preparation, C-6 Stereochemistry, and Reactions. J. Am. Chem. Soc. 85, 2643-2652 (1963).
32. L. Bernardi, R. de Castiglione, V. Colonna, P. Masi, R. Mazzoleni, Tetracycline derivatives I. Esters of 5-oxytetracyclines: chemistry and biological activity. Farmaco Sci. 29, 902-909 (1974).
33. H. B. Atakan, M. Cornaglia, L. Mouchiroud, J. Auwerx, M. A. M. Gijs, Automated high-content phenotyping from the first larval stage till the onset of adulthood of the nematode Caenorhabditis elegans. Lab Chip. 19, 120-135 (2018).
34. L. F. S. Bastos, A. Angusti, M. C. Vilaça, L. A. Merlo, E. B. Nascimento, L. T. S. Rocha, A. M. Godin, A. G. R. Solano, S. Jarussophon, E. A. Nunan, Y. Konishi, M. M. Coelho, A novel non-antibacterial, non-chelating hydroxypyrazoline derivative of minocycline inhibits nociception and oedema in mice. Br J Pharmacol. 155, 714-721 (2008).
35. M. Nelson, W. Hillen, R. A. Greenwald, Eds., Tetracyclines in Biology, Chemistry and Medicine (Birkhäuser Basel, Basel, 2001; http://link.springer.com/10.1007/978-3-0348-8306-1).
36. M. Moriyama, T. Koshiba, T. Ichinohe, Influenza A virus M2 protein triggers mitochondrial DNA-mediated antiviral immune responses. Nat Commun. 10, 4624 (2019).
37. H. K. Chung, J. T. Kim, H.-W. Kim, M. Kwon, S. Y. Kim, M. Shong, K. S. Kim, H.-S. Yi, GDF15 deficiency exacerbates chronic alcohol- and carbon tetrachloride-induced liver injury. Scientific Reports. 7, 17238 (2017).
38. J.-Y. Zhang, X.-M. Wang, X. Xing, Z. Xu, C. Zhang, J.-W. Song, X. Fan, P. Xia, J.-L. Fu, S.-Y. Wang, R.-N. Xu, X.-P. Dai, L. Shi, L. Huang, T.-J. Jiang, M. Shi, Y. Zhang, A. Zumla, M. Maeurer, F. Bai, F.-S. Wang, Single-cell landscape of immunological responses in patients with COVID-19. Nature Immunology. 21, 1107-1118 (2020).
39. N. A. Khan, J. Nikkanen, S. Yatsuga, C. Jackson, L. Wang, S. Pradhan, R. Kivelä, A. Pessia, V. Velagapudi, A. Suomalainen, mTORC1 Regulates Mitochondrial Integrated Stress Response and Mitochondrial Myopathy Progression. Cell Metab. 26, 419-428.e5 (2017).
40. S. Zaim, J. H. Chong, V. Sankaranarayanan, A. Harky, COVID-19 and Multiorgan Response. Current Problems in Cardiology. 45, 100618 (2020).
41. F. Supek, M. Boŝnjak, N. Ŝkunca, T. Ŝmuc, REVIGO summarizes and visualizes long lists of gene ontology terms. *PLoS One.* **6,** e21800 (2011).
42. Y. Steuerman, M. Cohen, N. Peshes-Yaloz, L. Valadarsky, O. Cohn, E. David, A. Frishberg, L. Mayo, E. Bacharach, I. Amit, I. Gat-Viks, Dissection of Influenza Infection In Vivo by Single-Cell RNA Sequencing. Cell Systems. 6, 679-691.e4 (2018).
43. I. Angelidis, L. M. Simon, I. E. Fernandez, M. Strunz, C. H. Mayr, F. R. Greiffo, G. Tsitsiridis, M. Ansari, E. Graf, T.-M. Strom, M. Nagendran, T. Desai, O. Eickelberg, M. Mann, F. J. Theis, H. B. Schiller, An atlas of the aging lung mapped by single cell transcriptomics and deep tissue proteomics. Nature Communications. 10, 963 (2019).
44. M. Jovanovic, M. S. Rooney, P. Mertins, D. Przybylski, N. Chevrier, R. Satija, E. H. Rodriguez, A. P. Fields, S. Schwartz, R. Raychowdhury, M. R. Mumbach, T. Eisenhaure, M. Rabani, D. Gennert, D. Lu, T. Delorey, J. S. Weissman, S. A. Carr, N. Hacohen, A. Regev, Dynamic profiling of the protein life cycle in response to pathogens. Science. 347 (2015), doi:10.1126/science.1259038.
45. M. Kissig, J. Ishibashi, M. J. Harms, H.-W. Lim, R. R. Stine, K.-J. Won, P. Seale, PRDM16 represses the type I interferon response in adipocytes to promote mitochondrial and thermogenic programing. The EMBO Journal. 36, 1528-1542 (2017).
46. J. Hadjadj, N. Yatim, L. Barnabei, A. Corneau, J. Boussier, N. Smith, H. Péré, B. Charbit, V. Bondet, C. Chenevier-Gobeaux, P. Breillat, N. Carlier, R. Gauzit, C. Morbieu, F. Pène, N. Marin, N. Roche, T.-A. Szwebel, S. H. Merkling, J.-M. Treluyer, D. Veyer, L. Mouthon, C. Blanc, P.-L. Tharaux, F. Rozenberg, A. Fischer, D. Duffy, F. Rieux-Laucat, S. Kernéis, B. Terrier, Impaired type I interferon activity and inflammatory responses in severe COVID-19 patients. Science. 369, 718-724 (2020).
47. I.-E. Galani, N. Rovina, V. Lampropoulou, V. Triantafyllia, M. Manioudaki, E. Pavlos, E. Koukaki, P. C. Fragkou, V. Panou, V. Rapti, O. Koltsida, A. Mentis, N. Koulouris, S. Tsiodras, A. Koutsoukou, E. Andreakos, Untuned antiviral immunity in COVID-19 revealed by temporal type I/III interferon patterns and flu comparison. Nature Immunology. 22, 32-40 (2021).
48. I. Chopra, M. Roberts, Tetracycline Antibiotics: Mode of Action, Applications, Molecular Biology, and Epidemiology of Bacterial Resistance. Microbiol Mol Biol Rev. 65, 232-260 (2001).
49. A. P. West, G. S. Shadel, Mitochondrial DNA in innate immune responses and inflammatory pathology. Nature reviews. Immunology. 17, 363-375 (2017).
50. M. W. Beilharz, J. M. Cummins, A. L. Bennett, Protection from lethal influenza virus challenge by oral type 1 interferon. Biochemical and Biophysical Research Communications. 355, 740-744 (2007).
51. S. Davidson, S. Crotta, T. M. McCabe, A. Wack, Pathogenic potential of interferon αβ in acute influenza infection. Nature Communications. 5, 3864 (2014).
52. N. Wang, Y. Zhan, L. Zhu, Z. Hou, F. Liu, P. Song, F. Qiu, X. Wang, X. Zou, D. Wan, X. Qian, S. Wang, Y. Guo, H. Yu, M. Cui, G. Tong, Y. Xu, Z. Zheng, Y. Lu, P. Hong, Retrospective Multicenter Cohort Study Shows Early Interferon Therapy Is Associated with Favorable Clinical Responses in COVID-19 Patients. Cell Host Microbe. 28, 455-464.e2 (2020).
53. J. Major, S. Crotta, M. Llorian, T. M. McCabe, H. H. Gad, S. L. Priestnall, R. Hartmann, A. Wack, Type I and III interferons disrupt lung epithelial repair during recovery from viral infection. Science. 369, 712-717 (2020).
54. R. Channappanavar, A. R. Fehr, R. Vijay, M. Mack, J. Zhao, D. K. Meyerholz, S. Perlman, Dysregulated Type I Interferon and Inflammatory Monocyte-Macrophage Responses Cause Lethal Pneumonia in SARS-CoV-Infected Mice. Cell Host & Microbe. 19, 181-193 (2016).
55. C. Balducci, G. Forloni, Doxycycline for Alzheimer's Disease: Fighting β-Amyloid Oligomers and Neuroinflammation. Front Pharmacol. 10 (2019), doi:10.3389/fphar.2019.00738.
56. Lindeman Jan H.N., Abdul-Hussien Hazem, van Bockel J. Hajo, Wolterbeek Ron, Kleemann Robert, Clinical Trial of Doxycycline for Matrix Metalloproteinase-9 Inhibition in Patients With an Abdominal Aneurysm. Circulation. 119, 2209-2216 (2009).
57. M. E. Ritchie, B. Phipson, D. Wu, Y. Hu, C. W. Law, W. Shi, G. K. Smyth, limma powers differential expression analyses for RNA-sequencing and microarray studies. Nucleic acids research. 43, e47 (2015).
58. G. Yu, L. G. Wang, Y. Han, Q. Y. He, clusterProfiler: an R package for comparing biological themes among gene clusters. Omics : a journal of integrative biology. 16, 284-7 (2012).
59. Babraham Bioinformatics - FastQC A Quality Control tool for High Throughput Sequence Data, (available at https://www.bioinformatics.babraham.ac.uk/projects/fastqc/).
60. A. Dobin, C. A. Davis, F. Schlesinger, J. Drenkow, C. Zaleski, S. Jha, P. Batut, M. Chaisson, T. R. Gingeras, STAR: ultrafast universal RNA-seq aligner. Bioinformatics. 29, 15-21 (2013).
61. M. D. Robinson, D. J. McCarthy, G. K. Smyth, edgeR: a Bioconductor package for differential expression analysis of digital gene expression data. Bioinformatics. 26, 139-140 (2010).
62. D. Risso, J. Ngai, T. P. Speed, S. Dudoit, Normalization of RNA-seq data using factor analysis of control genes or samples. Nature Biotechnology. 32, 896-902 (2014).
63. M. Conti, T. Delvienne, S. Loric, in Mitochondrial Dysfunction Caused by Drugs and Environmental Toxicants, Y. Will, J. A. Dykens, Eds. (John Wiley & Sons, Inc., Hoboken, NJ, USA, 2018; http://doi.wiley.com/10.1002/9781119329725.ch15), pp. 249-263.
64. J. Kim, R. Gupta, L. P. Blanco, S. Yang, A. Shteinfer-Kuzmine, K. Wang, J. Zhu, H. E. Yoon, X. Wang, M. Kerkhofs, H. Kang, A. L. Brown, S.-J. Park, X. Xu, E. Z. van Rilland, M. K. Kim, J. I. Cohen, M. J. Kaplan, V. Shoshan-Barmatz, J. H. Chung, VDAC oligomers form mitochondrial pores to release mtDNA fragments and promote lupus-like disease. Science. 366, 1531-1536 (2019).
65. U. Valcavi, N. Pacini, G. Campanella, Pyrazole derivatives of tetracycline and chlortetracycline. Gazz. Chim. Ital. 93 (7), 916-28 (1963).

## Claims

1. A compound of formula (I) or formula (II) 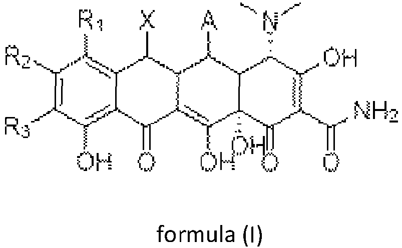 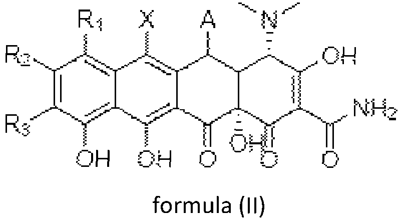
or a pharmaceutically acceptable salt thereof for use in treating or preventing a respiratory viral disease or viral infection,
wherein within formula (I) or (II)
(A)
A is H or OH,
X is CH₃, and
R₁ and R₂ are H and R₃ is a linear or branched C3 to C8 alkyl, and is preferably a branched C3 to C8 alkyl; or
(B)
A is H or OH,
X is CH₃, and
R₂ is H and R₁ and R₃ are a heteroaryl, wherein the heteroatom is preferably N and/or the heteroaryl is preferably substituted with one or two F.

2. The compound for use of claim 1, wherein R₃ is isopropyl, t-butyl, or t-pentyl, and is preferably t-butyl.

3. The compound for use of claim 1, wherein R₁ and R₃ are both bis-3-pyridine-2,6-di-fluor.

4. The compound for use of claim 1, wherein the respiratory viral disease or viral infection is caused by an influenza virus, coronavirus, bocavirus, rhinovirus, metapneumovirus, respiratory syncytial virus (RSV), parainfluenza viruses or respiratory adenoviruses.

5. The compound for use of claim 4, wherein the influenza virus is an influenza A or B and is preferably an influenza A virus.

6. The compound for use of claim 4, wherein the coronavirus is a betacoronavirus.

7. The compound for use of claim 6, wherein the betacoronavirus is selected from SARS-CoV-2, MERS-CoV, SARS-CoV-1, OC43, and HKU1, and is most preferably SARS-CoV-2.

8. The compound for use of any one of claims 1 to 7, wherein the compound is to be administered at a dosage of 0.0001 to 400 mg per kilogram of body weight per day and/or 1 to 800 mg per day.

9. The compound for use of any one of claims 1 to 8, wherein the compound displays a minimum inhibitory concentration (MIC) for *E. coli* of at least 8 µg/mL and/or for *P*. *aeruginosa* of at least 4 µg/mL.

10. The compound for use of any one of claims 1 to 9, wherein the compound is capable of activating an adaptive mitochondrial stress response.

## Patentansprüche

1. Verbindung der Formel (I) oder Formel (II) 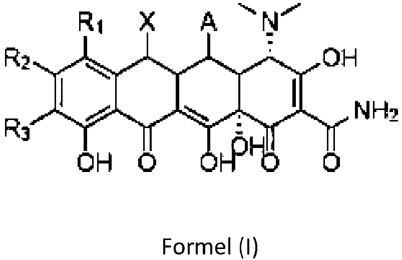 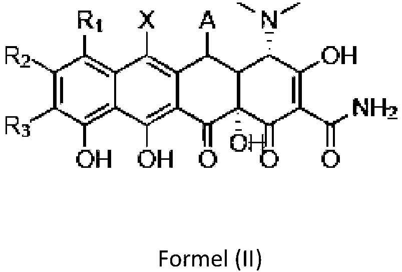
oder ein pharmazeutisch verträgliches Salz davon zur Verwendung in der Behandlung oder Vorbeugung einer viralen Erkrankung oder viralen Infektion der Atemwege, wobei in Formel (I) oder (II)
(A)
A H oder OH ist,
X CH₃ ist, und
R₁ und R₂ H sind und R₃ ein lineares oder verzweigtes C3 bis C8 Alkyl ist, und vorzugsweise ein verzweigtes C3 bis C8 Alkyl ist; oder
(B)
A H oder OH ist,
X CH₃ ist, und
R₂ H ist und R₁ und R₃ ein Heteroaryl sind, wobei das Heteroatom vorzugsweise N ist und/oder das Heteroaryl vorzugsweise mit einem oder zwei F substituiert ist.

2. Verbindung zur Verwendung nach Anspruch 1, wobei die Gruppen A, X, R₁ und R₂ wie im Merkmal (A) definiert sind und R₃ Isopropyl, t-Butyl, oder t-Pentyl ist, und vorzugsweise *t*-Butyl ist.

3. Verbindung zur Verwendung nach Anspruch 1, wobei die Gruppen A, X und R₂ wie im Merkmal (B) definiert sind, und R₁ und R₃ beide bis-3-Pyridin-2,6-difluor sind.

4. Verbindung zur Verwendung nach Anspruch 1, wobei die virale Erkrankung oder virale Infektion der Atemwege von einem Influenzavirus, Coronavirus, Bocavirus, Rhinovirus, Metapneumovirus, respiratorischen Syncytial-Virus (RSV), Parainfluenzavirus oder von respiratorischen Adenoviren verursacht wird.

5. Verbindung zur Verwendung nach Anspruch 4, wobei das Influenzavirus ein Influenza A oder B und vorzugsweise ein Influenza A Virus ist.

6. Verbindung zur Verwendung nach Anspruch 4, wobei das Coronavirus ein Betacoronavirus ist.

7. Verbindung zur Verwendung nach Anspruch 6, wobei das Betacoronavirus aus SARS-CoV-2, MERS-CoV, SARS-CoV-1, OC43, und HKU1 ausgewählt ist und vorzugsweise SARS-CoV-2 ist.

8. Verbindung zur Verwendung nach einem der Ansprüche 1 bis 7, wobei die Verbindung in einer Dosierung von 0,0001 bis 400 mg pro Kilogramm Körpergewicht pro Tag und/oder 1 bis 800 mg pro Tag zu verabreichen ist.

9. Verbindung zur Verwendung nach einem der Ansprüche 1 bis 8, wobei die Verbindung eine minimale Hemmkonzentration (MIC) für *E. coli* von mindestens 8 µg/mL und/oder für *P. aeroginoso* von mindestens 4 µg/mL aufweist.

10. Verbindung zur Verwendung nach einem der Ansprüche 1 bis 9, wobei die Verbindung eine adaptive mitochondriale Stressreaktion aktivieren kann.

## Revendications

1. Composé de formule (I) ou de formule (II) 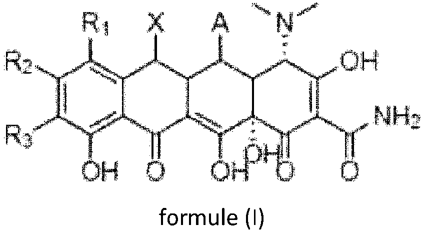 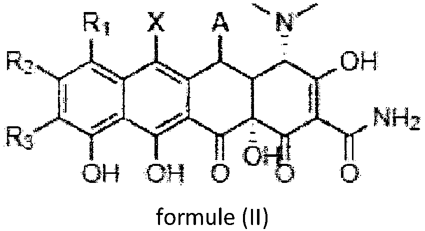
ou un sel pharmaceutiquement acceptable de celui-ci, pour son utilisation dans le traitement ou la prévention d'une infection virale ou d'une maladie virale respiratoire,
dans lequel, dans la formule (1) ou (II)
(A)
A est H ou OH,
X est CH₃, et
R₁ et R₂ sont H et R₃ est un alkyle en C₃ à C₈ linéaire ou ramifié, et est de préférence un alkyle en C₃ à C₈ ramifié ; ou
(B)
A est H ou OH,
X est CH₃, et
R₂ est H et R₁ et R₃ sont un hétéroaryle, dans lequel l'hétéroatome est de préférence N et/ou l'hétéroaryle est de préférence substitué par un ou deux F.

2. Composé pour son utilisation selon la revendication 1, dans lequel les groupes A, X, R₁ et R₂ sont tels que définis au point (A), et R₃ est un isopropyle, un t-butyle ou un t-pentyle, et est de préférence un t-butyle.

3. Composé pour son utilisation selon la revendication 1, dans lequel les groupes A, X et R₂ sont tels que définis au point (B), et R₁ et R₃ sont tous les deux un bis-3-pyridine-2,6-difluor.

4. Composé pour son utilisation selon la revendication 1, dans lequel l'infection virale ou la maladie virale respiratoire est causée par un virus grippal, un coronavirus, un bocavirus, un rhinovirus, un métapneumovirus, un virus respiratoire syncytial (VRS), des virus paragrippaux ou des adénovirus respiratoires.

5. Composé pour son utilisation selon la revendication 4, dans lequel le virus grippal est un virus grippal A ou B et est de préférence un virus grippal A.

6. Composé pour son utilisation selon la revendication 4, dans lequel le coronavirus est un bêta-coronavirus.

7. Composé pour son utilisation selon la revendication 6, dans lequel le bêta-coronavirus est choisi parmi SARS-CoV-2, MERS-CoV, SARS-CoV-1, OC43, et HKU1, et est de préférence SARS-CoV-2.

8. Composé pour son utilisation selon l'une quelconque des revendications 1 à 7, dans lequel le composé est destiné à être administré à une dose de 0,0001 à 400 mg par kilogramme de poids corporel et par jour et/ou de 1 à 800 mg par jour.

9. Composé pour son utilisation selon l'une quelconque des revendications 1 à 8, dans lequel le composé présente une concentration minimale inhibitrice (CMI) pour *E. coli* d'au moins 8 µg/mL et/ou pour *P. aeruginosa* d'au moins 4 µg/mL.

10. Composé pour son utilisation selon l'une quelconque des revendications 1 à 9, dans lequel le composé est capable d'activer une réponse adaptative au stress mitochondrial.
